# EUROPEAN PATENT APPLICATION

(11) **EP 2 062 590 A1**
(43) Date of publication of application: **27.05.2009**
(21) Application number: 08021733.4
(22) Date of filing: 22.09.2004
(51) Int. Cl.: A61K 38/16, C07K 14/18, C07K 16/10, C12N 15/12, G01N 33/576

(54) **Peptide derived from Hepatitis C virus**

(30) Priority: 22.09.2003 JP 2003330258
(62) Divisional of application: 04773486.8
(71) Applicant: Green Peptide Co., Ltd., Kurume City Fukuoka 839-0864 (JP)
(72) Inventor: Itoh, Kyogo, Miyaki-gun Saga 841-0205 (JP); Yamada, Akira, Ogoori-shi Fukuoka 838-0103 (JP); Sata, Michio, Fukuoka-shi Fukuoka 814-0002 (JP)
(74) Representative: Vossius & Partner

(57) **Abstract**

Disclosed is a peptide derived from hepatitis C virus comprising an HLA-binding motif in its sequence and being recognized by an antibody detected in a patient with hepatitis C virus (HCV)infection. The peptide of the present invention is useful in diagnosing hepatitis C virus, treating hepatitis C, and predicting the prognosis of hepatitis C.

## Description

### Technical Field

The present invention relates to a hepatitis C virus (HCV) peptide, which is useful for diagnosing hepatitis C virus infection and treating hepatitis C.

More particularly, the invention relates to an HCV peptide, a polypeptide comprising an HCV peptide, a nucleotide encoding an HCV peptide or the polypeptide, an antibody or a substance with an antibody-like activity that recognizes the peptide or polypeptide, a vector comprising the nucleotide, a method of inducing a cytotoxic T cell with the HCV peptide or the polypeptide, a method of detecting hepatitis C virus and a method of diagnosing, preventing or treating hepatitis C, a method of predicting the prognosis of hepatitis C using the HCV peptide, the polypeptide, the nucleotide, or the antibody or the substance with an antibody-like activity, as well as a pharmaceutical composition for treating hepatitis C comprising the HCV peptide, the polypeptide, the nucleotide, or the antibody or the substance with an antibody-like activity.

### Background Art

Hepatitis caused by viruses includes hepatitis A mainly caused by oral infection with virus and hepatitis B caused by infection through blood, and hepatitis C mainly caused by infection through blood transfusion. The hepatitis C is one of the diseases that may develop into a chronic disease resulting in hepatic cirrhosis or liver cancer at an extremely high risk.

Hepatitis C virus (HCV) is a single-stranded RNA virus that belongs to the flavivirus family, and 2 million or more in Japan, and 170 million around the world are said to be infected with hepatitis C virus.

A combination therapy with interferon and ribavirin has been developed for treating hepatitis C, however, it is effective only in 30 to 40% of patients, and effective therapy is not available for the majority of the patients. Accordingly, there has been a strong social demand to immediately develop diagnostic and therapeutic tools which are safe, effective and simple to use at low costs.

There is evidence showing that both cellular and humoral immune responses play a primary role in inhibiting HCV infection (WO 89/04669). A number of studies have been conducted over the past 10 years to find an HCV peptide with high immunogenicity, and many HCV peptides have been discovered that can induce a cellular or humoral immune response (Hunziker et al., Mol Immunol. 2001 Dec; 38(6): 475-84). However, any of these peptides is not clinically effective. There is no effective immunotherapeutic means for preventing and treating hepatitis C even at present, which is believed to be primary due to the poor immunogenicity of these peptides.

In addition, the present inventors have already reported that an antigenic peptide recognized by some cytotoxic T lymphocytes (CTLs) has an ability to induce both cellular and humoral immune responses both in vitro and in vivo (Gohara et al., J Immunother. 2002 Sep-Oct; 25(5): 439-44, Mine et al., Clin Cancer Res. 2001 Dec; 7(12): 3950-62, Tanaka et al., J Immunother. 2003 Jul-Aug; 26(4): 357-66, Mine et al., Cancer Sci. 2003 Jun; 94(6): 548-56).

An HCV peptide recognized by both cellular and humoral immune responses is expected to have higher immunogenicity than a peptide recognized only by a cellular immune response.

A new approach to meet such a social demand is to identify an HCV peptide which can induce both cellular and humoral immunity, since accumulating findings demonstrated that such a peptide would have much higher immunogenicity than a peptide inducing only a humoral immunity.

Thus, it will be advantageous to identify such a peptide and to examine whether such a peptide is a candidate for a novel therapeutic and diagnostic tool for HCV infection.

Accordingly, the present inventors have examined whether IgG reactive to a peptide recognized by HLA-A2-and HLA-A24-restricted cytotoxic T lymphocytes (CTLs) can be detected in the serum of a patient infected with HCV, and found that IgG specific for certain CTL epitopes is detected in the majority of the patients infected with HCV regardless of their HLA class I type or the HCV genotype. From this result, they found that such a peptide may lead to novel preventive and therapeutic tools utilizing the peptide.

Accordingly, an object of the invention is to provide an HCV peptide which is recognized by cellular and humoral immunity and is highly immunogenic.

### Disclosure of the invention

The present invention provides a peptide derived from hepatitis C virus, comprising an HLA-binding motif in its sequence and is recognized by an antibody present in the blood of a patient infected with hepatitis C virus.

In a preferred embodiment, the peptide derived from hepatitis C virus has an amino acid sequence represented by any one of SEQ ID NOS: 1 to 8, 16, 20 and 38 of the sequence listing, or has an amino acid sequence having a homology of at least 70% with the amino acid sequence of the HCV-derived peptide, and has a property of being recognized by an HLA-A2- or HLA-A24-restricted cytotoxic T cell.

Another aspect of the invention provides a polypeptide comprising the peptide derived from hepatitis C virus. In a preferred embodiment, the invention also provides a polypeptide having an amino acid sequence having a homology of at least 70% with the amino acid sequence of the polypeptide, and a polypeptide further having a property of being recognized by an HLA-A2- or HLA-A24-restricted cytotoxic T cell.

Yet another aspect of the invention provides a nucleotide encoding the peptide derived from hepatitis C virus or the polypeptide.

Still another aspect of the invention provides an antibody or a substance with an antibody-like activity capable of immunologically recognizing the peptide derived from hepatitis C virus or the polypeptide.

In still another aspect, the invention provides a vector comprising the above-mentioned nucleotide.

Still another aspect of the invention provides a method of inducing a cytotoxic T cell by using the nucleotide encoding the peptide derived from hepatitis C virus or the polypeptide.

In still another aspect, the invention provides a method of detecting hepatitis C virus using the peptide derived from hepatitis C virus, polypeptide, nucleotide or antibody or substance with an antibody-like activity.

Another aspect of the invention provides a method of diagnosing a disease associated with HCV infection such as hepatitis C using the peptide derived from hepatitis C virus, polypeptide, nucleotide or antibody or substance with an antibody-like activity.

In another aspect, the invention provides a method of treating a disease associated with HCV infection such as hepatitis C using the peptide derived from hepatitis C virus, polypeptide, nucleotide or antibody or substance with an antibody-like activity.

In still another aspect, the invention provides a pharmaceutical composition comprising as an active ingredient the peptide derived from hepatitis C virus, polypeptide, nucleotide or antibody or substance with an antibody-like activity. In a preferred embodiment, the pharmaceutical composition is a hepatitis C virus vaccine.

In another aspect, the invention provides a method of predicting the prognosis of a disease associated with HCV infection such as hepatitis C using the peptide derived from hepatitis C virus, polypeptide, nucleotide or antibody or substance with an antibody-like activity.

In still another aspect, the invention provides a kit for diagnosing hepatitis C or predicting the prognosis of hepatitis C comprising the peptide derived from hepatitis C virus, polypeptide, nucleotide or antibody or substance with an antibody-like activity.

### Brief Description of the Drawings

Fig. 1 is a graph showing the results of measuring the IgG level in the blood of a patient infected with HCV by the ELISA method according to the present invention.
Fig. 2 is a graph showing the results of measuring the IgG level in the blood of patients infected with HCV by the ELISA method according to the present invention.
Fig. 3 is a graph showing the experimental results of absorption and elution of a representative example of an antibody reactive to C-35 peptide present in the serum of the patient shown in Fig. 1 according to the present invention.
Fig. 4 is a graph showing the results of CTL induction with 6 types of HLA-A24 binding peptides (Nos. 3, 12, 13, 25, 32 and 38) derived from HCV according to the present invention.
Fig. 5 is a graph showing the results of CTL induction with HLA-A2 binding peptides (Nos. 40 to 57) derived from HCV according to the present invention.
Fig. 6 is a graph showing representative results of the cytotoxic activity of peptide-stimulated PBMC in a ⁵¹Cr release assay according to the present invention.
Fig. 7 is a graph showing representative results of the cytotoxic activity of peptide-stimulated PBMC in a ⁵¹Cr release assay according to the present invention.
Fig. 8 is a graph showing the HLA class I restriction of the cytotoxicity of peptide-stimulated PBMC in an inhibition assay with an anti-CD8 monoclonal antibody according to the present invention.
Fig. 9 is a graph showing detection of anti- peptide IgG in the sera of patients infected with HCV.
Fig. 10 is a graph showing detection of anti-peptide IgG in the sera of patients infected with HCV.
Fig. 11 is a graph showing the results of adsorption test analyzing the specificity of anti-peptide IgG present in the sera of patients infected with HCV.
Fig. 12 is a graph showing the results of elution test analyzing the specificity of anti-peptide IgG in the sera of patients infected with HCV.
Fig. 13 is a graph showing IFN-γ production by peptide-stimulated PBMC.
Fig. 14 is a graph showing IFN-γ production by peptide-stimulated PBMC.
Fig. 15 is a graph showing the cytotoxicity of peptide-stimulated PBMC.
Fig. 16 is a graph showing the cytotoxicity of peptide-stimulated PBMC.
Fig. 17 is a graph showing the CTL activity of peptide-stimulated PBMC against NS5A-expressing cells.
Fig. 18 is a graph showing the CTL activity of peptide-stimulated PBMC against NS5A-expressing cells.
Fig. 19 is a graph showing the CTL activity of peptide-stimulated PBMC against NS5A-expressing cells.
Fig. 20 is a graph showing the specificity of the activity of anti-NS5A-2132 IgG.
Fig. 21 is a graph showing the results of a cell growth inhibition assay using anti-NS5A-2132 IgG.
Fig. 22 is a graph showing the results of assaying the ADCC activity of anti-NS5A-2132 IgG.
Fig. 23 is a graph showing the detection of an anti-C-35 antibody and an anti-NS5A-2132 antibody in patients with various diseases.
Fig. 24 is a graph showing the HLA or HCV genotype restriction of anti-peptide antibodies.
Fig. 25 is a graph showing the HLA or HCV genotype restriction of anti-peptide antibodies.
Fig. 26 is a graph showing the results of measuring the level of anti-NS5A-2132 antibody in the sera of patients.
Fig. 27 is a graph showing the results of measuring the level of anti-NS5A-2132 IgG in the sera of patients.
Fig. 28 is a graph showing the levels of anti-NS5A antibody and HCV RNA in the sera of patients.
Fig. 29 is a graph showing the detection of an anti-C35 antibody and an anti-NS5A-2132 antibody in a cohort study.
Fig. 30 is a graph showing the detection of an anti-NS5A-2132 antibody in a cohort study.
Fig. 31 is a graph showing the results of measuring the IgG level in the blood of patients infected with HCV by a Luminex assay according to the present invention.
Fig. 32 is a graph showing the results of measuring the IgG level in the blood of patients infected with HCV by a Luminex assay according to the present invention.
Fig. 33 shows the course of a treatment of hepatitis C using peptides of the present invention.
Fig. 34 shows the course of a treatment of hepatitis C using peptides of the present invention.

### Detailed Description of the Invention

The peptide derived from hepatitis C virus of the invention is an HCV peptide, which contains the HLA-binding motif in its sequence and is recognized by an antibody present in the blood of a patient infected with hepatitis C virus.

In addition, the HCV peptide according to the invention contains an HLA-binding motif, i.e., an HLA-A2- or HLA-A24-binding motif in its sequence. This HCV peptide is also characterized by having a high immunogenicity as well as being recognized by cellular and humoral immunity.

Examples of the HCV peptide include those peptides listed in Tables 1 and 3. One of the particularly preferred HCV peptides of the invention is a peptide having the HLA-A2-binding motif with the following amino acid sequence:

### C-35: YLLPRRGPRL (SEQ ID NO: 1).

### In a patient,

IgG antibody reactive with this peptide is detected with significant proportion in patients infected with HCV with the detection ratio of 93% and the specificity for HCV infection of 100%. Further, particularly preferred HCV peptides of the invention are peptides having the HLA-A24-binding motif with any of the following amino acid sequences:
NS5A-2132: RYAPACKPL (SEQ ID NO: 2);
E2-488: HYAPRPCGI (SEQ ID NO: 3);
E1-213: VYEAADMIM (SEQ ID NO: 4);
NS3-1081: VYHGAGSKTL (SEQ ID NO: 5);
C-176: IFLLALLSCL (SEQ ID NO: 6);
C-173: SFSIFLLALL (SEQ ID NO: 7);
EYVLLLFLL (SEQ ID NO: 8);
PYIEQGMQL (SEQ ID NO: 16);
IFTITKILL (SEQ ID NO: 20); and
SFAIKWEYVL (SEQ ID NO: 38).
In particular, NS5A-2132 is able to induce both cellular and humoral immunity in many HLA-A24-positive patients.

The HCV peptide according to the invention may be a peptide having a homology of at least 70%, preferably 80% or more, more preferably 90% or more, with the amino acid sequence of any of the peptides listed above.

The HCV peptide according to the invention may further comprise a peptide having a property of being recognized by an HLA-A2- or HLA-A24-restricted cytotoxic T cell (CTL). This type of antigenic peptide capable of binding to HLA is generated by intracellular degradation of an antigenic protein produced in a cell, and has a sequence motif for each HLA type. A cytotoxic T cell (CTL) recognizes a complex of the antigenic peptide and HLA and causes damage in an HCV-infected cell.

The polypeptide according to the invention comprises in its sequence the amino acid sequence of the peptide of the invention as described above, but the total number of amino acids is not particularly limited. In addition, in the polypeptide of the invention, the amino acid sequence other than that of the peptide are located at the N-terminal side and/or the C-terminal side or both sides of the amino acids of the peptide. Therefore, such a polypeptide has substantially the same function and effect as those of the above-mentioned peptide. When the term "peptide" is used herein, it should be construed that "polypeptide" is also included in the term unless otherwise specified.

The peptide having an amino acid sequence as described above can be obtained by a standard chemical synthesis, by an enzymatic degradation of a protein molecule, or by a recombinant DNA technique using a host transformed so as to express a nucleotide sequence encoding a desired amino acid sequence.

In the case where the desired peptide is produced by a chemical synthetic method, it can be produced by a technique which is well known per se and is commonly used in standard peptide chemistry. For example, the peptide can be synthesized by a solid phase synthetic method with a peptide synthesizer. A crude peptide obtained in this way can be purified by any of the purification method commonly used in protein chemistry, such as salting out, ultrafiltration, reverse-phase chromatography, ion-exchange chromatography and affinity chromatography.

In the case where the desired peptide is produced by a recombinant DNA technique, the desired peptide can be obtained by, for example, integrating a DNA fragment encoding a desired amino acid sequence synthesized as described above into an appropriate expression vector, transforming a microorganism or an animal cell using this expression vector and then culturing the thus obtained transformant. The expression vector that may be used in the invention includes a plasmid, a viral vector, etc., which are well known in the art.

To transform a host cell with an expression vector in the peptide-producing technique described above, any of well known methods may be appropriately selected and used, such as a calcium chloride method, a calcium phosphate co-precipitation method, a DEAE-dextran method, a Lipofectin method or an electroporation method. The peptide can be purified by any of the above-mentioned purification methods from a cell extract or a culture supernatant collected from the culture medium.

A nucleotide, which is one of the other aspects of the invention, includes an oligonucleotide or a polynucleotide encoding the above-mentioned peptide derived from hepatitis C virus or the above-mentioned polypeptide. The nucleotide may include a ribonucleotide and a deoxynucleotide. The nucleotide may be modified by a method known in the art. Examples of the modification of the nucleotide include labeling, methylation, "caps", substitution of one or more of naturally occurring nucleotides with an analog, and intranucleotide modification known in the art. Examples of the intranucleotide modification include nonionic modification (e.g., a methyl phosphonate, a phosphotriester, a phosphoramidate, etc.), ionic modification (e.g., a phosphorothioate, a phosphorodithioate etc.), modification of incorporating a pendant moiety such as a protein (e.g., a nuclease, a toxin, an antibody, a signal peptide etc.), modification with a chelating agent (e. g. , a metal, boron etc.).

The above-mentioned nucleotide sequence allows for provision of a peptide or a polypeptide sequence of an HCV antigen encoded by the genome of HCV, as well as a peptide useful for a diagnostic test or as a component of a vaccine.

Once the nucleotide of the invention is obtained, it becomes possible to construct a nucleotide probe and a peptide useful for diagnosing a disease associated with HCV infection or for screening HCV infection in the blood or a blood preparation. Based on this nucleotide sequence, a DNA oligomer having, for example, 24 to 30 nucleotides or even more may be synthesized. The nucleotide can also be used as a probe for detecting HCV RNA in the serum of a subject or for screening the presence of HCV in the blood or blood products.

Furthermore, the nucleotide sequence allows for design and production of an HCV-specific peptide useful as a reagent for testing the presence of an antibody against HCV. An antibody raised against a purified peptide having this sequence may be used for detecting an HCV antigen in a subject infected with HCV and in blood products which might have been prepared from the blood of a person infected with HCV.

In another aspects of the invention, the antibody includes a chimeric antibody, a modified antibody, a monovalent antibody, Fab, F(ab')₂, Fab', a single chain Fv (scFV) protein and a single domain antibody, which are reactive to the peptide derived from hepatitis C virus or the polypeptide. These antibodies can immunologically recognize the peptide or the polypeptide.

In another aspects of the invention, a vector is a construct comprising the nucleotide and can transform a selected host cell to express heterologous coding sequences in the host. The expression vector may be either a cloning vector or an integration-type vector.

The cloning vector may include, for example, a plasmid and a virus (such as an adenovirus vector). The cloning vector is a replicon that can transform a host cell and has an ability to replicate in the cell (e.g., a plasmid, a chromosome, a virus, a cosmid and the like).

An integration vector is a vector that does not serve as a replicon in a host cell, but has an ability to integrate its contents into a replicon (typically a chromosome) in the host to stably transform the host.

Another aspect of the invention provides a method of inducing a cytotoxic T cell targeted to an HCV-infected cell using the peptide. The induction method of the invention may comprise, for example, contacting the peptide with a cell expressing HLA-A2 to present the peptide on the HLA-A2 molecule, stimulating a T cell with the cell presenting the peptide with HLA-A2, and inducing the T cell into CTL. The HLA-A2-expressing cell to be used in this method may be collected from the blood of an HCV patient, however, it can also be prepared by introducing a gene encoding HLA-A2 into a cell that does not express HLA-A2. Thus, the peptide according to the invention is useful for detection and diagnosis of HCV infection and is also useful as a pharmaceutical composition, such as a vaccine for the treatment of a disease associated with hepatitis C virus.

CTL as induced in this way will target and attack a cell infected with HCV, since a CTL may be also as a pharmaceutical composition for a cell therapy.

In another aspect of the invention, the peptide derived from hepatitis C virus, polypeptide, nucleotide and/or antibody or substance with an antibody-like activity is useful for detecting or diagnosing HCV infection.

The detection of HCV and the diagnosis of a disease associated with HCV can be carried out by, for example, detecting the presence of the peptide, detecting the presence or amount of the corresponding nucleotide sequence, determining the biological distribution of the peptide in an individual and/or determining the amount of the peptide present in a specimen derived from an individual. Such a method may utilize the interaction and/or reactivity with the nucleotide sequence encoding the peptide. In other words, the detection of HCV and the diagnosis of a disease associated with HCV may be effected by assaying the peptide as a marker. The assay can be carried out by a well known method by, for example, an antigen-antibody reaction system, an enzymatic reaction system, and a PCR reaction system.

Monitoring of the effect of the treatment and the prediction the prognosis of a disease associated with HCV can be carried out by monitoring the concentration of an antibody reactive to the peptide in the blood of a subject having a disease associated with HCV. It is particularly preferred to measure the level of anti-C-35 IgG or anti-NS5A-2132 IgG in the blood of a subject.

In still another aspect of the invention, the pharmaceutical composition includes a vaccine. The vaccine comprises the peptide derived from hepatitis c virus, the polypeptide and/or the nucleotide, and may suitably contain a pharmaceutically acceptable adjuvant and/or carrier. An adjuvant can enhance the immune response, and may include incomplete Freund's adjuvant or aluminum hydroxide gel. The carrier may include, for example, a diluent such as PBS or distilled water, physiological saline.

The pharmaceutical composition of the invention can be administered orally, parenterally or transdermally depending on the administration form, for example, via intravenous administration or subcutaneous administration. Examples of the dosage form include tablets, granules, soft capsules, hard capsules, liquids, oils, and emulsions. The dose of such a pharmaceutical composition can suitably selected depending on the symptoms of the patient to be treated. In general, the amount of the peptide may range from 0.1 to 10 mg per day for an adult, and the composition may be administered once every several days or several months.

The entire contents of all patents and reference documents explicitly cited in the present specification are incorporated herein by reference. In addition, the entire contents described in the specification and drawings of Japanese Patent Application No. 2003-330258 to which this application claims priority are also incorporated herein by reference.

### Examples

The invention will be described in more detail with reference to Examples below. These Examples are provided to illustrate the invention more specifically, but does not limit the scope of the invention.

The abbreviations for amino acids used in the following Examples are as follows.

A denotes alanine, C, cysteine; D, aspartic acid; E, glutamic acid; F, phenylalanine; G, glycine; H, histidine; I, isoleucine; K, lysine; L, leucine; M, methionine; P, proline; R, arginine; S, serine; T, threonine; V, valine; and Y, tyrosine.

### Example 1: Detection of IgG reactive to HCV peptide in the sera of patients infected with HCV

### Peptide

Synthetic peptides having an HLA-A2-binding motif or an HLA-A24-binding motif from a conserved region of HCV genotype 1b protein were used in this study (Table 1). A peptide derived from HIV having an HLA-A2-binding motif was used as a negative control. All of these peptides were purchased from a commercially supplier. The purity was analyzed by reverse-phase high-pressure liquid chromatography and found to be 90% or higher.

**Table 1**

| No | Peptide name | alias | Region | Sequence | SEQ ID NO | HCV patients (n=12) | | Healthy donors (n = 10) | |
|---|---|---|---|---|---|---|---|---|---|
| | | | | | | Positive | | Positive | |
| | | | | | | n | % | n | % |
| 3 | 1bA24-2132 | NS5A-2132 | NS5A | RYAPACKPL | 2 | 12 | 100 | 1 | 10 |
| 4 | 1bA24-717 | E2-717 | E2 | EYVLLLFLL | 8 | 2 | 17 | 0 | 0 |
| 5 | 1bA24-1100 | NS3-1100 | NS3 | MYTNVDQDL | 9 | 0 | 0 | 0 | 0 |
| 6 | 1bA24-1773 | NS4A-1773 | NS4A | QYLAGLSTL | 10 | 0 | 0 | 0 | 0 |
| 7 | 1bA24-790 | E2-790 | E2 | LYGVWPLLL | 11 | 0 | 0 | 0 | 0 |
| 8 | 1bA24-1031 | NS3-1031 | NS3 | AYSQQTRGL | 12 | 0 | 0 | 0 | 0 |
| 9 | 1bA24-834 | NS2-834 | NS2 | HYKLFLARL | 13 | 0 | 0 | 0 | 0 |
| 10 | 1bA24-1292 | NS3-1292 | NS3 | TYATYGKFL | 14 | 1 | 8 | 0 | 0 |
| 11 | 1bA24-1266 | NS3-1266 | NS3 | AYMSKAHGI | 15 | 6 | 50 | 0 | 0 |
| 12 | 1bA24-488 | E2-488 | E2 | HYAPRPCGI | 3 | 9 | 75 | 0 | 0 |
| 13 | 1bA24-213 | E1-213 | E1 | VYEAADMIM | 4 | 6 | 50 | 0 | 0 |
| 14 | 1bA24-1716 | NS4B-1716 | NS4B | PYIEQGMQL | 16 | 1 | 8 | 0 | 0 |
| 15 | 1bA24-1767 | NS4B-1767 | NS4B | NFISGIQYL | 17 | 0 | 0 | 0 | 0 |
| 16 | 1bA24-910 | NS2-910 | NS2 | PYFVRAHGL | 18 | 0 | 0 | 0 | 0 |
| 17 | 1bA24-1727 | NS4A-1727 | NS4A | QFKQKAIGL | 19 | 3 | 25 | 0 | 0 |
| 18 | 1bA24-885 | NS2-885 | NS2 | IFTITKILL | 20 | 5 | 42 | 0 | 0 |
| 19 | 1bA24-135 | C-135 | C | GYIPIVGAPL | 21 | 1 | 8 | 0 | 0 |
| 20 | 1bA24-631 | E2-631 | E2 | MYVGGVEHRL | 22 | 0 | 0 | 0 | 0 |
| 21 | 1bA24-932 | NS2-932 | NS2 | HYVQMALMKL | 23 | 5 | 42 | 0 | 0 |
| 22 | 1bA24-947 | NS2-947 | NS2 | TYVYDHLTPL | 24 | 7 | 58 | 0 | 0 |
| 23 | 1bA24-1854 | NS4B-1854 | NS4B | GYGAGVAGAL | 25 | 2 | 17 | 0 | 0 |
| 24 | 1bA24-1243 | NS3-1243 | NS3 | AYAAQGYKVL | 26 | 2 | 17 | 0 | 0 |
| 25 | 1bA24-1081 | NS3-1081 | NS3 | VYHGAGSKTL | 5 | 6 | 50 | 0 | 0 |
| 26 | 1bA24-787 | E2-787 | E2 | AYALYGVWPL | 27 | 2 | 17 | 0 | 0 |
| 27 | 1bA24-617 | E2-617 | E2 | HYPCTVNFTI | 28 | 4 | 33 | 0 | 0 |
| 28 | 1bA24-1417 | NS3-1417 | NS3 | YYRGLDVSVI | 29 | 2 | 17 | 0 | 0 |
| 29 | 1bA24-2146 | NS5A-2146 | NS5A | TFLVGLNQYL | 30 | 2 | 17 | 0 | 0 |
| 30 | 1bA24-822 | NS2-822 | NS2 | VFVGLILLTL | 31 | 4 | 33 | 0 | 0 |
| 31 | 1bA24-1556 | NS3-1556 | NS3 | EFWESVFTGL | 32 | 0 | 0 | 0 | 0 |
| 32 | 1bA24-176 | C-176 | C | IFLLALLSCL | 6 | 7 | 58 | 0 | 0 |
| 33 | 1bA24-837 | NS2-837 | NS2 | LFLARLIWWL | 33 | 0 | 0 | 0 | 0 |
| 34 | 1bA24-848 | NS2-848 | NS2 | YFITRAEAHL | 34 | 1 | 8 | 1 | 10 |
| 35 | 1bA24-1792 | NS4B-1792 | NS4B | AFTASITSPL | 35 | 0 | 0 | 0 | 0 |
| 36 | 1bA24-1990 | NS5A-1990 | NS5A | DFKTWLQSKL | 36 | 1 | 8 | 0 | 0 |
| 37 | 1bA24-2121 | NS5A-2121 | NS5A | FFTEVDGVRL | 37 | 1 | 8 | 0 | 0 |
| 38 | 1bA24-173 | C-173 | C | SFSIFLLALL | 7 | 7 | 58 | 0 | 0 |
| 39 | 1bA24-711 | E2-711 | E2 | SFAIKWEYVL | 38 | 1 | 8 | 0 | 0 |
| 40 | 1bA2-35 | C-35 | C | YLLPRRGPRL | 1 | 12 | 100 | 0 | 0 |
| 41 | 1bA2-132 | C-132 | C | DLMGYIPLV | 39 | 0 | 0 | 0 | 0 |
| 42 | 1bA2-178 | C-178 | C | LLALLSCLTV | 40 | 1 | 8 | 0 | 0 |
| 43 | 1bA2-220 | E1-220 | E1 | ILHTPGCV | 41 | 3 | 25 | 0 | 0 |
| 44 | 1bA2-363 | E2-363 | E2 | SMVGNWAKV | 42 | 0 | 0 | 0 | 0 |
| 45 | 1bA2-401 | E2-401 | (HVR) | SLLAPGAKQNV | 43 | 0 | 0 | 3 | 30 |
| 46 | 1bA2-1073 | NS3-1073 | NS3 | CINGVCWTV | 44 | 0 | 0 | 0 | 0 |
| 47 | 1bA2-1169 | NS3-1169 | NS3 | LLCPAGHAV | 45 | 1 | 8 | 2 | 20 |
| 48 | 1bA2-1287 | NS3-1287 | NS3 | KLVALGINAV | 46 | 1 | 8 | 0 | 0 |
| 49 | 1bA2-1406 | NS3-1406 | NS3 | TGAPVTYSTY | 47 | 1 | 8 | 0 | 0 |
| 50 | 1bA2-1789 | NS4B-1789 | NS4B | SLMAFTAAV | 48 | 0 | 0 | 0 | 0 |
| 51 | 1bA2-1807 | NS4B-1807 | NS4B | LLFNILGGWV | 49 | 0 | 0 | 0 | 0 |
| 52 | 1bA2-1851 | NS4B-1851 | NS4B | ILAGYGAGV | 50 | 0 | 0 | 1 | 10 |
| 53 | 1bA2-2252 | NS5A-2252 | NS5A | ILDSFDPLV | 51 | | 0 | 0 | 0 |
| 54 | 1bA2-2692 | NS5B-2692 | NS5B | RLIVFPDLGV | 52 | 1 | 8 | 0 | 0 |
| 55 | 1bA2-2727 | NS5B-2727 | NS5B | GLQDCTMLV | 53 | 2 | 17 | 1 | 10 |
| 56 | 1bA2-150 | C-150 | C | ALAHGVRAL | 54 | 0 | 0 | 1 | 10 |
| 57 | 1bA2-168 | C-168 | C | NLPGCSFSI | 55 | 0 | 0 | 1 | 10 |
| 60 | 1bA24-2422 | NS5B-2422 | NS5B | SYTWTGALI | 56 | 0 | 0 | 0 | 0 |
| 61 | 1bA24-2482 | NS5B-2482 | NS5B | HYRDVLKEM | 57 | 3 | 25 | 1 | 10 |
| 62 | 1bA24-2990 | NS5B-2990 | NS5B | WFMWCLLLL | 58 | 7 | 58 | 0 | 0 |
| 63 | 1bA24-789 | E2-789 | E2 | AFYGVWPLL | 59 | 0 | 0 | 0 | 0 |
| 64 | 1bA24-1727 | NS4B-1727 | NS4B | QFKQKALGL | 60 | 1 | 8 | 0 | 0 |
| 65 | 1bA24-2613 | NS5B-2613 | NS5B | QYSPGQRVEF | 61 | 0 | 0 | 0 | 0 |
| 66 | 1bA24-1727 | NS4B-1727 | NS4B | QFKQKALGLL | 62 | 3 | 25 | 0 | 0 |

### The cut-off value is 1.83.

**Table 2**

| | No. | 40 |
|---|---|---|
| | | prevalence |
| Diseases associated with HCV infection | 60 | 56 (93.3%) |
| Subjects infected with hepatitis B virus | 24 | 0 (0%) |
| Hepatitis B virus vaccine recipients | 10 | 0 (0%) |
| Healthy donors | 27 | 0 (0%) |
| Patients with an autoimmune disease | 22 | 0 (0%) |
| Subjects infected with HTLV-I | 10 | 8 (80%) |
| Subjects infected with HIV | 3 | 3 (100%) |
| Total | 156 | 67 |
| | | |
| | | C-35 |
| Sensitivity | | 93.3% |
| Sensitivity | | 88.5% |
| OD ratio | | |
| | | P<0.001 |

The detection rate was calculated based on the number of positive patients with above the cutoff value (mean ± 3 SD).

The detection rates of NS-5A-2132 and C-35 are 0.202 and 0.13, respectively.

Statistical analysis was carried out by the χ²-test.

### ELISA

The level of the peptide-specific IgG in the serum was measured by ELISA. First, each peptide was dissolved in dimethyl sulfoxide (DMSO) and stored at -80°C. The peptide was diluted with 0.1 M sodium carbonate/sodium bicarbonate solution containing disuccinimidyl suberate (DSS) as a crosslinker. An ELISA plate was coated with 20 µg/well of peptide at 4°C overnight. The wells were washed 3 times with 0.05% Tween 20/PBS (PBST), and the plate was blocked with Block Ace (registered trademark) and left overnight at 4°C. Plasma or serum samples were diluted in 1:100, 1:200 and 1:400 with 0.05% Tween 20/Block Ace, and 100 µl of each of the samples was added to each well. After the samples were incubated at 37°C for 2 hours, the plate was washed 9 times with PBST, 100 µl of a 1:1000 diluted rabbit anti-human IgG (γ-chain specific) was added to each well, and incubated at 37°C for 2 hours. The plate was washed 9 times with PBST, and 100 µl of 1:100 diluted mouse anti-rabbit IgG conjugated to a horseradish peroxidase-dextran polymer was added to each well, and then the plate was incubated at room temperature for 40 minutes. After the plate was washed, 100 µl of a tetramethylbenzidine substrate solution was added to each well. The reaction was stopped by the addition of 2.0 M phosphoric acid. The cutoff value was calculated as the mean ± 3 SD of the optical density values of the control obtained from the healthy donors. Absorption and elution of peptide-specific IgG

One hundred microliters of a plasma or serum sample diluted with 0.05% Tween 20/Block Ace (registered trademark) was added to each well and allowed to be absorbed to the peptide (20 µg/well) coated on the well of the plate at 37°C for 2 hours. After this procedure was repeated 3 times, the peptide-specific IgG level in the supernatant was measured by ELISA. The antibody bound to the peptide-coated plate in the initial adsorption step was eluted with 30 µl/well of 5 M NaCl/50 mM citrate buffer (pH 3.0) (neutralized with 0.05% Tween 20/Block Ace containing 1.0 M Tris-HCl (pH 7.5)), and the peptide-specific IgG level in the eluted fraction was measured by ELISA.

### Assay for peptide-specific CTL

A peptide-specific CTL was detected by a method commonly used in this technical field. PBMCs were added to each well of a round-bottomed 96-well microculture plate at 1 x 10⁵ cells per well, and the cells were incubated in 200 µl of a medium with 10 µM peptide. The composition of this medium is as follows: 45% RPMI-1640, 45% AIM-V (registered trademark), 10% fetal calf serum (FCS), 100 U/ml interleukin 2 (IL-2), and 1 mM MEM non-essential amino acid solution. On days 3, 6 and 9 of culture, one half of each culture solution was removed and replaced with a fresh medium containing a corresponding peptide (20 µg/ml). On day 12 of culture, the cultured cells were collected and assayed for the interferon gamma (IFN-γ) production in response to CIR-A2402 cell or T2 previously pulsed with the corresponding peptide or an HIV peptide as a negative control. Four wells were used for each peptide and the assay was carried out twice. The background IFN-γ production in response to the HIV peptide was subtracted from the obtained data value. For the inhibition assay, a peptide-reactive CTL was purified using a CD8 isolation kit, and assayed for the peptide-specific IFN-γ production in the presence of 20 µ/ml of any of an anti-HLA class I (W6/32, IgG2a) monoclonal antibody, an anti-CD8 (Nu-Ts/c, IgG2a) monoclonal antibody and an anti-HLA class II (H-DR-1, IgG2a) monoclonal antibody. The background IFN-γ production was subtracted from the obtained data value.

### Statistics

The values are expressed as the mean ± SD. A statistical analysis was carried out using the Mann-Whitney U-test and the chi-square test, and a P value less than 0.05 was considered statistically significant.

### Detection of antibody against HCV peptide

The level of IgG that reacts with each of the 62 types of peptides was measured in the sera from 12 patients infected with HCV and 10 healthy donors (HDs) (Table 1). The prevalence of the antibody against each peptide is shown in Table 1. A plurality of peptides were found that highly reacted with IgG in the blood from the patients infected with HCV and rarely react with the sera from the healthy donors.

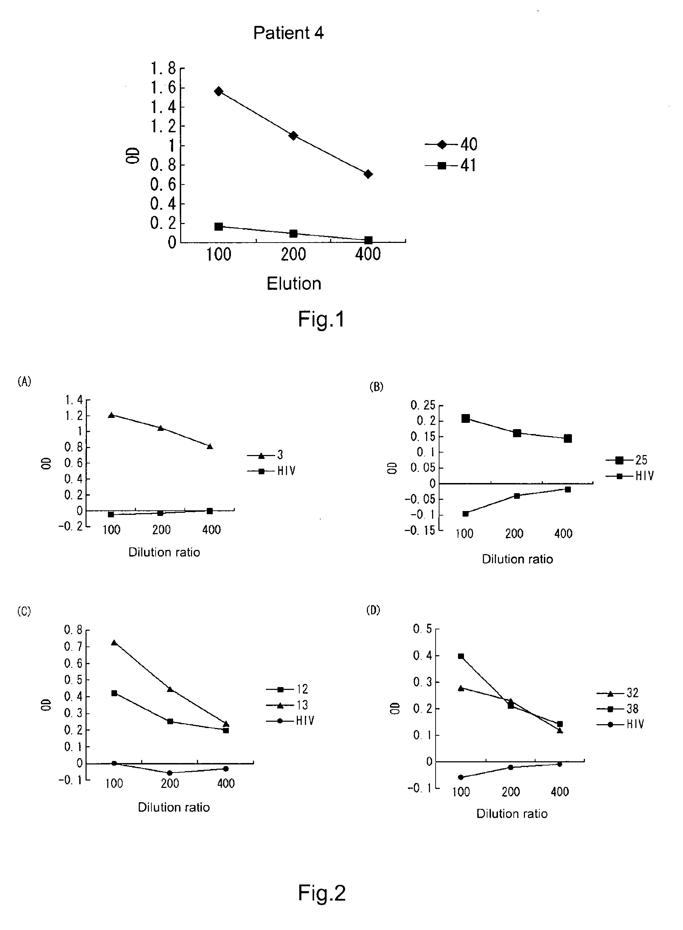

The peptides include No. 3: NS5A-2132 (corresponding to the peptide at positions 2132-2140 of HCV NS5A protein, hereinafter abbreviated in a similar fashion), No. 11: NS3-1266, No. 12: E2-488, No. 13: E1-213, No. 22: NS2-947, No. 25: NS3-1081, No. 32: C-176, No. 38: C-173, No. 40: C-35, and No. 62: NS5B-2990. Representative results of measuring IgG level in the blood from the patients infected with HCV by the ELISA method are shown in Figs. 1 and 2.

In the serum from the patient shown in Fig. 1, an IgG antibody against C-35 was detected.

In the sera from 4 patients shown in Fig. 2 (A) to (D), IgG antibodies reactive to NS5A-2132 (A), NS3-1081 (B), E2-488 and E1-213 (C) and C-176 and C-173 (D) were detected, respectively. The peptide specificity of those peptide-reactive antibodies was examined by absorption and elution tests.

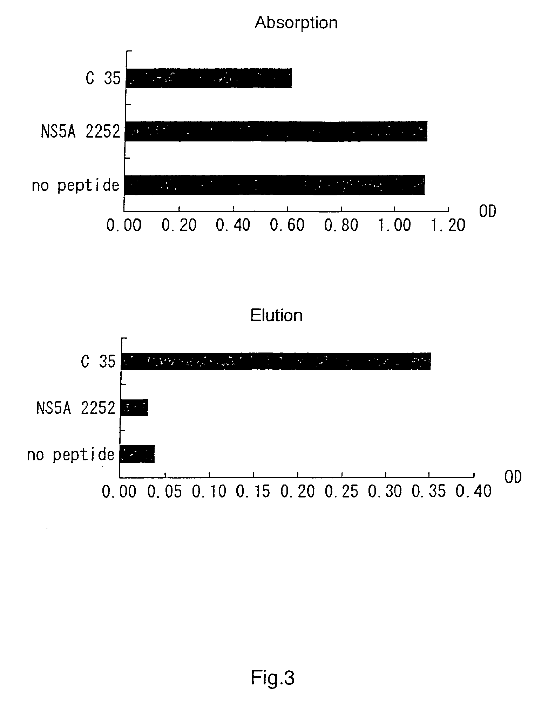

Fig. 3 shows a representative example of the experimental results of absorption and elution of an antibody reactive to C-35 peptide in the serum of the patient shown in Fig. 1. This antibody was absorbed by C-35, but not by an irrelevant peptide NS5A-2252 (upper graph of Fig. 3). Furthermore, the antibody was eluted from the C-35-adsorbed fraction (the lower graph of Fig. 3).

Among the antibodies listed in Table 1, the antibody reactive to C-35 peptide was detected at 100% in the patients infected with HCV and at 0% in the healthy donors, indicating that it has high specificity to HCV. The sequence of C-35 peptide of HCV has a partial homology with peptides of various viruses, such as tet protein (the amino acid sequence at positions 54-58) or env protein (amino acid sequences at positions 5-9, 158-162, and 717-727) of HIV-1. It also shows a homology with various proteins of HTLV-I (such as pol 724-755, rex 5-9, rex 310-313 and tax 70-74). Thus, the antibody reactive to C-35 peptide was further examined in a double-blind study of 156 cases in total, including 60 cases of diseases associated with HCV infection (22 cases of chronic hepatitis C, 20 cases of liver cirrhosis and 18 cases of liver cancer), 24 cases of subjects infected with hepatitis B virus, 10 cases of hepatitis B virus vaccine recipients, 27 cases of healthy donors, 22 cases of patients with an autoimmune disease, 10 cases of subjects infected with HTLV-I and 3 cases of subjects infected with HIV. The results are shown in Table 2. The HCV specificity and the detection rate of the antibody reactive to C-35 peptide were 88.5% and 93.3%, respectively.

### Induction of peptide-specific CTL activity

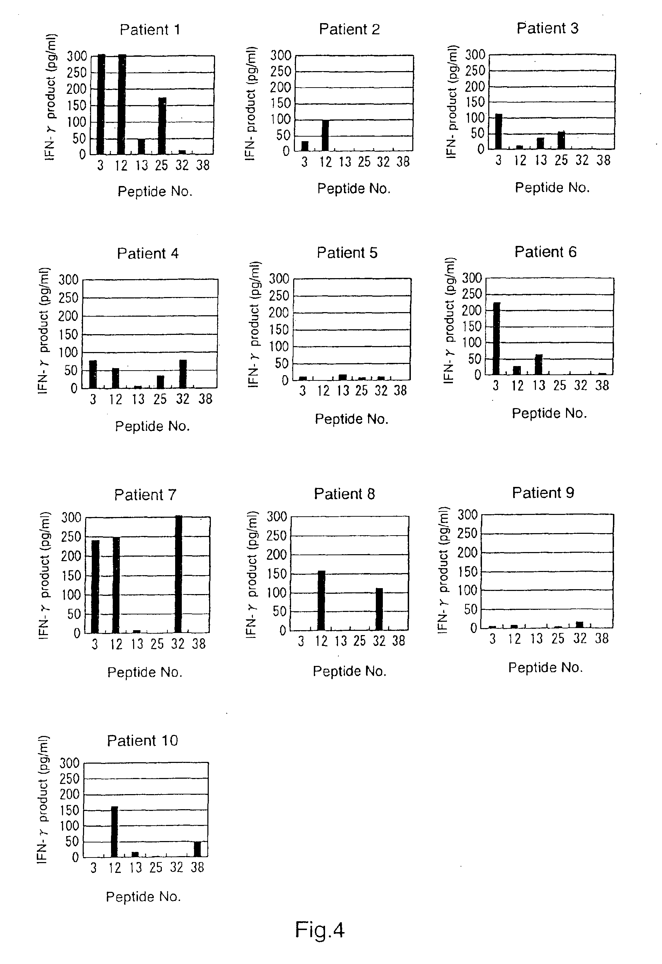

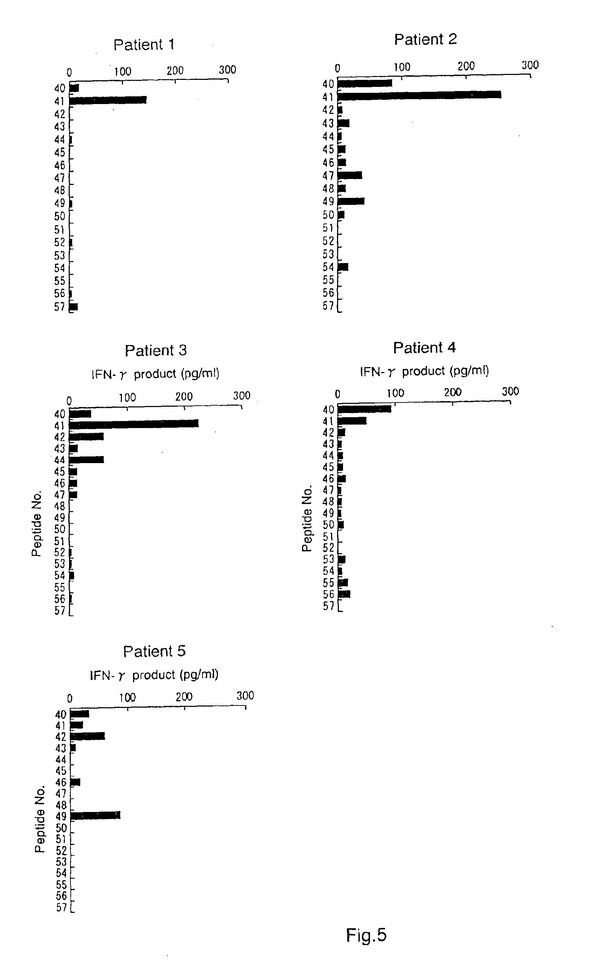

Six types of HLA-A24 binding peptides (Nos. 3, 12, 13, 25, 32 and 38) and 18 types of HLA-A2 binding peptides (Nos. 40 to 57) were incubated with peripheral blood mononuclear cells (PBMCs) obtained from HLA-A24⁺ or HLA-A2⁺ HCV-infected patients (5 patients in each group). As a control, the PBMCs were incubated with an HIV peptide. Then, the incubated PBMCs were tested for their ability to produce IFN-γ in response to CIR-A2402 cell or T2 cell pulsed with a corresponding peptide. Six types of HLA-A24 binding peptides (Nos. 3, 12, 13, 25, 32 and 38) could induce CTL from the PBMC obtained from 10 HLA-A24⁺ HCV patients (Fig. 4). Furthermore, CTL could be induced by the HLA-A2 binding peptides derived from HCV (Nos. 40 to 57) in the PBMC obtained from 5 HLA-A2⁺ HCV patients (Fig. 5).

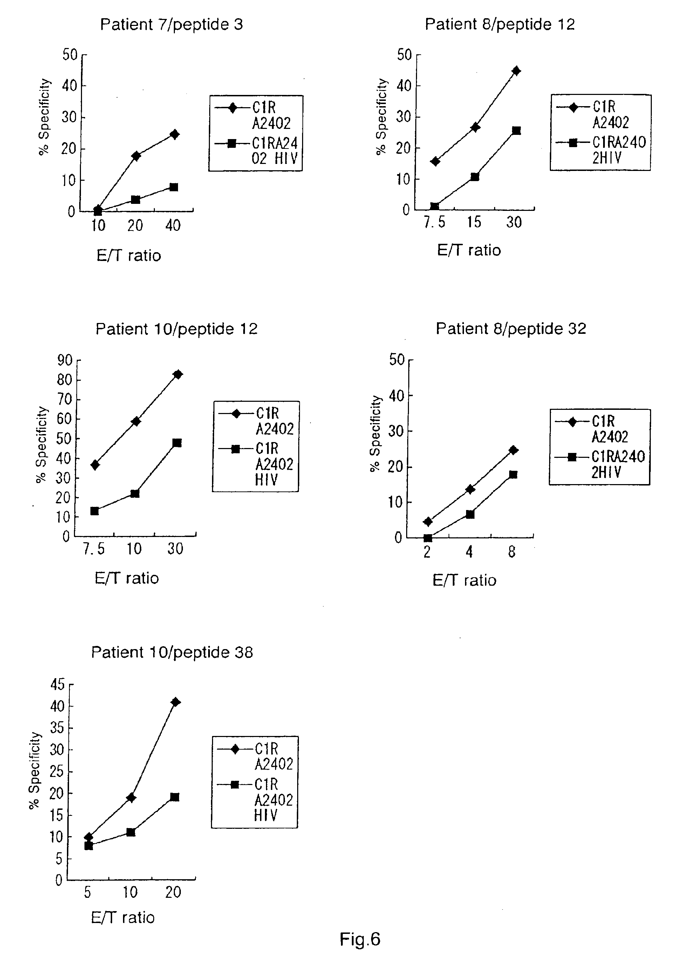

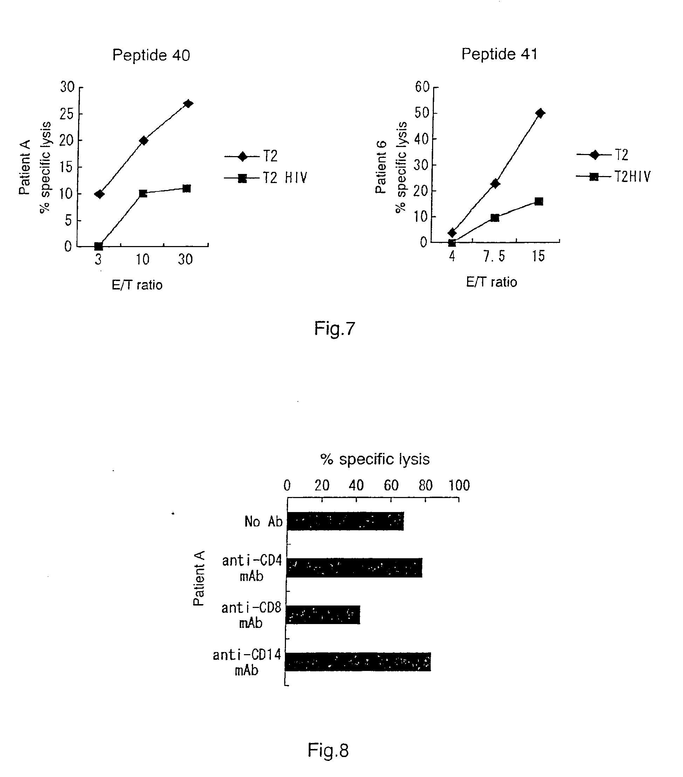

The cytotoxic activity of peptide- stimulated PBMC was measured by the ⁵¹Cr release assay. The representative results are shown in Figs. 6 and 7.

Fig. 6 shows the cytotoxic activity of CTL induced in the PBMC obtained from the HLA-A24⁺ HCV patients by the HLA-A24 binding peptides (Nos. 3, 12, 13, 25, 32 and 38) derived from HCV. The cytotoxic activity against C1R-A2402 cells loaded with the corresponding peptide (represented by ClRA2402 in the drawing) was significantly higher than that against the control C1R-A2402 cells loaded with the HIV peptide (represented by C1RA2402 HIV in the drawing).

Fig. 7 shows the cytotoxic activity of CTL induced in the peripheral blood PBMC obtained from the HLA-A2⁺ HCV patients by the HLA-A2 binding peptides (Nos. 40 and 41) derived from HCV. The cytotoxic activity against T2 cells loaded with the corresponding peptide (represented by T2 in the drawing) was significantly higher than that against the control T2 cells to loaded with the HIV peptide (represented by T2HIV in the drawing).

These PBMCs showed a significantly higher level of cytotoxic activity against the C1R-A2402 cell and the T2 cell preloaded with the corresponding peptide (except for an HIV peptide as a negative control).

The HLA class I restriction of the cytotoxicity was tested by the inhibition assay using an anti-CD8 monoclonal antibody (Fig. 8). Fig. 8 shows a representative example of inhibition of the CTL activity by the anti-CD8 antibody. The cytotoxicity of the CTL induced in the PBMC obtained from the HLA-A2⁺ HCV patient by the HLA-A2 binding peptide (No. 40) derived from HCV was inhibited by the anti-CD8 antibody, but not by an anti-CD4 antibody or the control anti-CD14 antibody.

### Example 2: Detection of IgG reactive to HCV peptide in the sera of HLA-A24⁺ HCV-infected patients

### Subject

The detection of IgG reactive to the HCV peptide was carried out more extensively in HLA-A24⁺ HCV-infected patients. Sixty HCV-1b⁺ patients were found to be seropositive for an anti-HCV antibody (Ab) indicated by the second generation or the third generation immunoassay test. The genotype of HCV contained in the sera of the patients was determined by RT-PCR. The patients were diagnosed at the time of serum sampling by biochemical analysis, echography, computed tomography and histological finding. The results of diagnosis are as follows: chronic hepatitis (CH, n = 24), liver cirrhosis (LC, n = 18) and hepatic cell carcinoma (HCC, n = 18). A negative control was serum samples from 20 healthy donors (HDs) who had normal hepatic function and no medical history of hepatitis virus.

### Assay of peptide and peptide-reactive antibody

Forty-four types of synthetic peptides that were derived from a highly conserved region of HCV-1b protein and selected based on a high binding score to HLA-A24 molecule (Bioinformatics and Molecular Analysis Section, NIH, Bethesda, MD) were used. A peptide derived from HIV that has an HLA-A24 binding motif (RYLRDQQLLGI (SEQ ID NO: 63)) served as a negative control. The binding scores and sequences of the peptides are shown in Table 3. For the screening of the reactivity of peptide to serum IgG, desalted grade of the peptides (> 70%) were purchased from BioSynthesis (Lewisville, TX) or SynPep (Dublin, CA). A purified peptide (> 90%) was used in the subsequent experiments. The plasma level of peptide-specific IgG was measured by an enzyme-linked immunosorbent assay (ELISA). The specificity of anti-peptide IgG in a plasma sample was tested by the absorption and elution of peptide-reactive IgG.

### Assay of peptide-specific CTL

Peptide-specific CTL precursor cells were detected by the method as described in Hida et al. (Cancer Immunol Immunother 2002; 51: 219-228). For an inhibition assay, 20 µg/ml of anti-HLA class I (W6/32, IgG2a), anti-CD8 (Nu-Ts/c, IgG2a), anti-CD4 (Nu-Th/i, IgGlb) and anti-HLA class II (H-DR-1, IgG) monoclonal antibodies were used.

### CTL activity of peptide-stimulated PBMC against cell transfected with NS5A gene

The cDNAs of NS5A, HLA-A2402 and HLA-A2601 were obtained by RT-PCR from Huh7 (NNU-50-1) cell, C1R-A2402 cell and KE4-CTL cell, respectively, and cloned into an expression vector pCR3.1 (Invitrogen, San Diego, CA). The Huh7 (NNU50-1) cell line is a replicon cell derived from a cell line infected with HCV-1b and expresses proteins from NS3 to NS5B (Kishine et al., Biochem Biophys Res Commun 2002; 293: 993-999). Then, 100 ng of NS5A, HLA-A2402 or NS5A and HLA-A2601 cDNA (as a negative control) were mixed in 100 µl of Opti-MEM (Invitrogen) containing 0.6 µl of Fugene6 (Roche Molecular Biochemicals, Indianapolis, IN) and incubated for 30 minutes. Then, 100 µl of the mixture was added to COS-7 cells (1 x 10⁴ cells) and incubated for 6 hours. One hundred microliters of RPMI-1640 medium containing 20% FCS was added to the mixture and COS-7 cells were cultured for 2 days, followed by the addition of NS5A 2132-2140-stimulated PBMCs (2 x 10⁵ cells/well). After incubation for 18 hours, 100 µl of the supernatant was removed and IFN-γ concentration was assayed by ELISA in triplicate. In order to prepare a stable transfectant cell line, either pCR3.1-NS5A or pCR3.1-HLA-A3101 (negative control) was transfected to C1R-A2402 cell by electroporation using a Gene Pulser (BioRAD, Richmond, CA). These cells were cultured for 30 to 40 days in the presence of geneticin (G418), and then geneticin resistant cells were collected. The expression of NS5A protein in the transfectant was assayed by Western blot analysis using an anti-NS5 polyclonal antibody (Abcam Limited, Cambridge, UK).

### Growth inhibition and antibody dependent cellular cytotoxicity (ADCC)

Huh7 cell line was incubated in a well of a flat-bottomed 96-well microculture plate in the presence of various sera for 24, 48 and 72 hours. After the incubation, Huh7 cells were counted with a Cell Count kit-8 (10 µl/well) (Dojindo, Japan). For ADCC assay, PBMCs newly isolated from a HLA-A24⁺ HD were pre-incubated for 1.5 hours in 10% FCS and RPMI-1640 medium containing inactivated serum (56°C, 30 minutes) from any of various patients and HD (as a negative control). C1R-A2402 cells or C1R cells (as a negative control) were incubated for 2 hours with NS5A 2132-2140 peptide (10 µM) and radiolabeled with Na₂⁵¹CrO₄ for 1.5 hours, washed and used as target cells. The peptide-stimulated PBMCs were incubated in a well of a round-bottomed 96-well microculture plate with the target cells at an effecter to the target cell (E/T) ratio of 40, 20 or 10 to 1. After incubation at 37°C for 6 hours, cell-free supernatant was collected and the ADCC activity was assayed (Shomura, et al., Br J Cancer 2004; 90: 1563-1571).

### Statistics

A statistical analysis was carried out using Student's t-test and the Mann-Whitney U-test. Values of P < 0.05 were considered statistically significant.

### Detection of humoral response to HCV-peptide

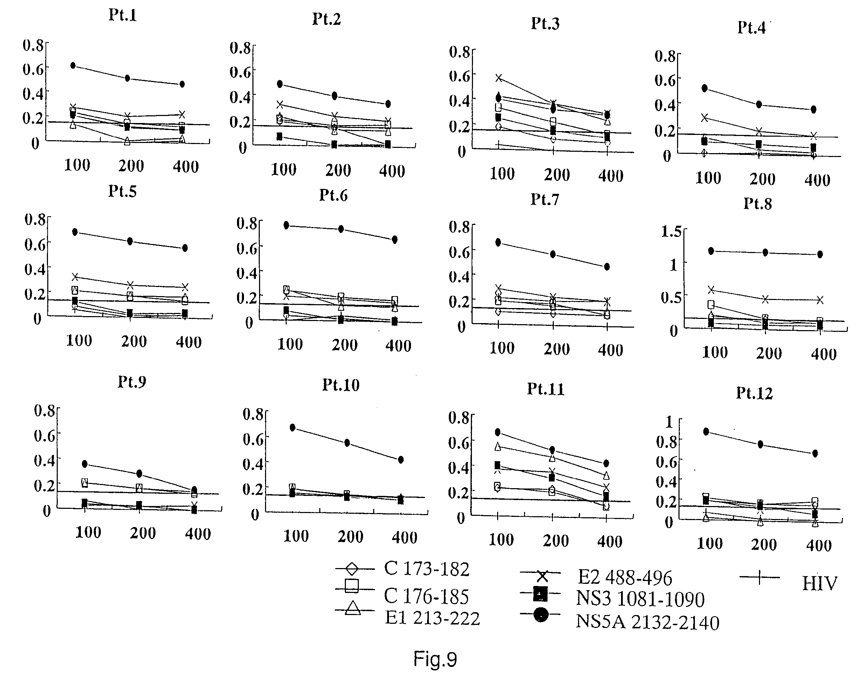

In order to screen for a humoral response to the peptides, the level of IgG reactive to each of the 44 types of peptides was assayed in the plasma of 12 patients infected with HCV-1b. The plasma of 10 HDs was used as a negative control. The level of peptide-reactive IgG was measured by ELISA for serially diluted plasma samples and reported by the values of absorbance (OD) of each sample. The cut-off value of these peptides at a plasma dilution of 1:100 was set at 0.18 OD (mean (0.08) + 2SD (0.05 x 2) of 10 HDs ). A significant level (> 0.18 OD at a plasma dilution of 1:100) of IgG reactive to NS5A-2132, C-176, E2-488, E1-213, C-173 and NS3-1081 peptides were detectable in the plasma of 12, 11, 10, 9, 8 and 5 patients among the 12 patients, respectively (Fig. 9). As expected, none of these peptides was reactive to the plasma from the 10 HDs. The reactivity of the peptides to peptide-specific IgG is summarized in Table 3.

**Table 3: HCV restricted peptides and reactivity to peptide-specific IgG ^{a)}**

| No. | Region | Peptide | SEQ ID NO | Reactivity to peptide-specific IgG | | |
|---|---|---|---|---|---|---|
| | | | | Binding score ^{b)} | Patients (n = 12) | HD (n = 10) |
| 1 | C 135-144 | GYGAGVAGAL | 25 | 504 | 1 | 0 |
| 2 | C 173-182 | SFSIFLLALL | 7 | 24 | 8 | 0 |
| 3 | C 176-185 | IFLLALLSCL | 6 | 36 | 11 | 0 |
| 4 | E1 213-221 | VYEAADMIM | 4 | 37.5 | 9 | 0 |
| 5 | E2 488-496 | HYAPRPCGI | 3 | 60 | 10 | 0 |
| 6 | E2 617-626 | HYPCTVNFTI | 28 | 75 | 2 | 0 |
| 7 | E2 631-640 | MYVGGVEHRL | 22 | 420 | 0 | 0 |
| 8 | E2 711-720 | SFAIKWEYVL | 38 | 20 | 1 | 0 |
| 9 | E2 717-725 | EYVLLLFLL | 8 | 432 | 2 | 0 |
| 10 | E2 787-796 | AYALYGVWPL | 27 | 200 | 2 | 0 |
| 11 | E2 789-797 | AFYGVWPLL | 59 | 20 | 0 | 0 |
| 12 | E2 790-798 | LYGVWPLLL | 11 | 200 | 0 | 0 |
| 13 | NS2 822-831 | VFVGLILLTL | 31 | 30 | 2 | 0 |
| 14 | NS2 834-842 | HYKLFLARL | 13 | 60 | 0 | 0 |
| 15 | NS2 837-846 | LFLARLIWWL | 33 | 36 | 0 | 0 |
| 16 | NS2 848-856 | YFITREAHL | 34 | 30 | 1 | 1 |
| 17 | NS2 885-893 | IFTITKILL | 20 | 20 | 3 | 0 |
| 18 | NS2 910-918 | PYFVRAHGL | 18 | 24 | 0 | 0 |
| 19 | NS2 932-941 | HYVQMALMKL | 23 | 396 | 3 | 0 |
| 20 | NS2 947-956 | TYVYDHLTPL | 24 | 300 | 2 | 0 |
| 21 | NS3 1031-1039 | AYSQQTRGL | 12 | 200 | 0 | 0 |
| 22 | NS3 1081-1090 | VYHGAGSKTL | 5 | 200 | 5 | 0 |
| 23 | NS3 1100-1108 | MYTNVDQDL | 9 | 336 | 0 | 0 |
| 24 | NS3 1243-1252 | AYAAQGYKVL | 26 | 200 | 2 | 0 |
| 25 | NS3 1266-1274 | AYMSKAHGI | 15 | 75 | 2 | 0 |
| 26 | NS3 1292-1300 | TYSTYGKFL | 14 | 200 | 1 | 0 |
| 27 | NS3 1417-1426 | YYRGLDVSVI | 29 | 50 | 2 | 0 |
| 28 | NS3 1556-1565 | EFWESVFTGL | 32 | 40.32 | 0 | 0 |
| 29 | NS4B 1716-1724 | PYIEQGMQL | 16 | 36 | 1 | 0 |
| 30 | NS4B 1727-1735 | QFKQKAIGL | 19 | 20 | 3 | 0 |
| 31 | NS4B 1727-1735 | QFKQKALGL | 60 | 20 | 1 | 0 |
| 32 | NS4B 1727-1736 | QFKQKALGLL | 62 | 20 | 1 | 0 |
| 33 | NS4B 1767-1775 | NFISGIQYL | 17 | 36 | 0 | 0 |
| 34 | NS4B 1773-1781 | QYLAGLSTL | 10 | 300 | 0 | 0 |
| 35 | NS4B 1792-1801 | AFTASITSPL | 35 | 28 | 0 | 0 |
| 36 | NS4B 1854-1863 | GYGAGVAGAL | 25 | 280 | 2 | 0 |
| 37 | NS5A 1990-1999 | DFKTWLQSKL | 36 | 26.4 | 1 | 0 |
| 38 | NS5A 2121-2130 | FFTEVDGVRL | 37 | 24 | 1 | 0 |
| 39 | NS5A 2132-2140 | RYAPACKPL | 2 | 480 | 12 | 1 |
| 40 | NS5A 2146-2156 | TFLVGNQYL | 30 | 43.2 | 2 | 0 |
| 41 | NS5B 2442-2451 | SYTWTGALI | 56 | 50 | 0 | 0 |
| 42 | NS5B 2482-2491 | HYRDVLKEM | 57 | 46.2 | 3 | 1 |
| 43 | NS5B 2613-2622 | QYSPGQRVEF | 61 | 132 | 0 | 0 |
| 44 | NS5B 2990-2998 | WFMWCLLLL | 58 | 30 | 3 | 0 |

| | | | | | | |
|---|---|---|---|---|---|---|
| a) The level of peptide-specific IgG in the sera of 12 HCV-1b⁺ patients was measured by ELISA.   The cut-off value was determined to be 0.18. b) The binging score of the peptide to HLA-A24 molecule was searched using Bioinformatics and Molecular Analysis Section, NIH, Bethesda, MD   (http://bimas.dcrt.nih.gov/molbio/hla_bind/). | | | | | | |

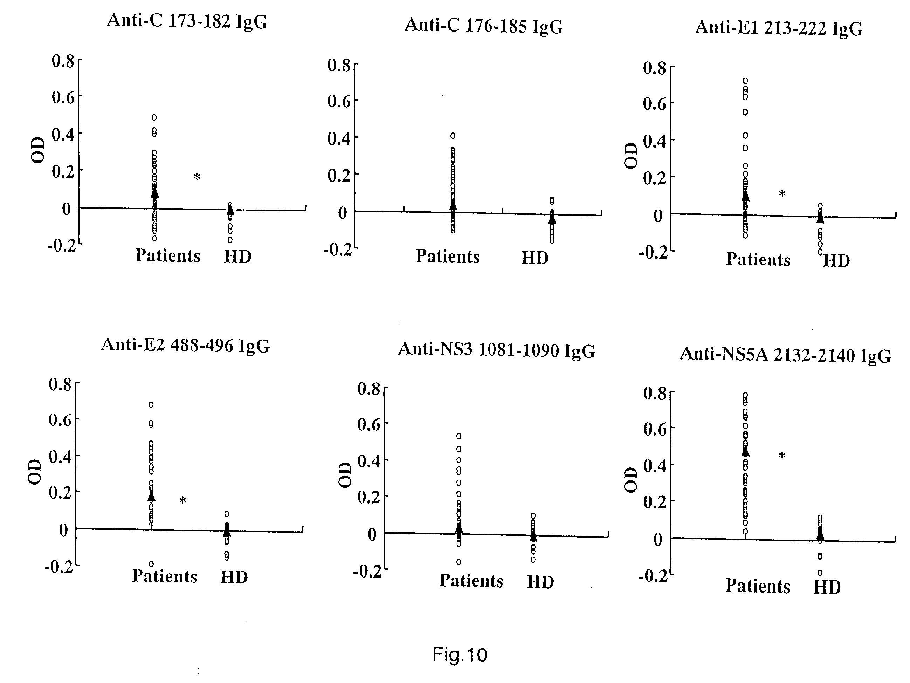

Each of the 6 types of the above peptides having a purity of 90% or more and the negative control peptide were tested for reactivity to each of the serum samples from 60 HCV-1b⁺ patients (CH, n = 24; LC, n = 18; and HCC, n = 18) and 20 HDs (Fig. 10).

The values for each of these samples at a dilution of 100:1 were plotted. The mean ± SD of IgG (OD value) was as follows: NS5A-2132 (0.48 ± 0.36), E2-488 (0.18 ± 0.19), E1-213 (0.10 ± 0.21), NS3-1081 (0.036 ± 0.14), C-176 (0.09 ± 0.14) and C-173 (0.04 ± 0.13). The asterisks indicate P < 0.05 in the Mann-Whitney U-test. The average level of IgG reactive to NS5A-2132, E2-488, E1-213 or C-173 was statistically significantly higher than that of HDs, however, it was not the case with NS3-1081 or C-176. IgG showing a significant level of reactivity (> 0.101 OD at a serum dilution of 1:100) to NS5A-2132, E2-488, E1-213, C-173, NS3-1081 and C-176 peptides was detected in the plasma of 57 (95%), 44 (73%), 28 (47%), 23 (38%), 21 (35%) and 17 (28%) patients of the 60 patients tested, respectively. On the contrary, a significant level of IgG could not be detected in any of the sera of 20 HDs (Fig. 10).

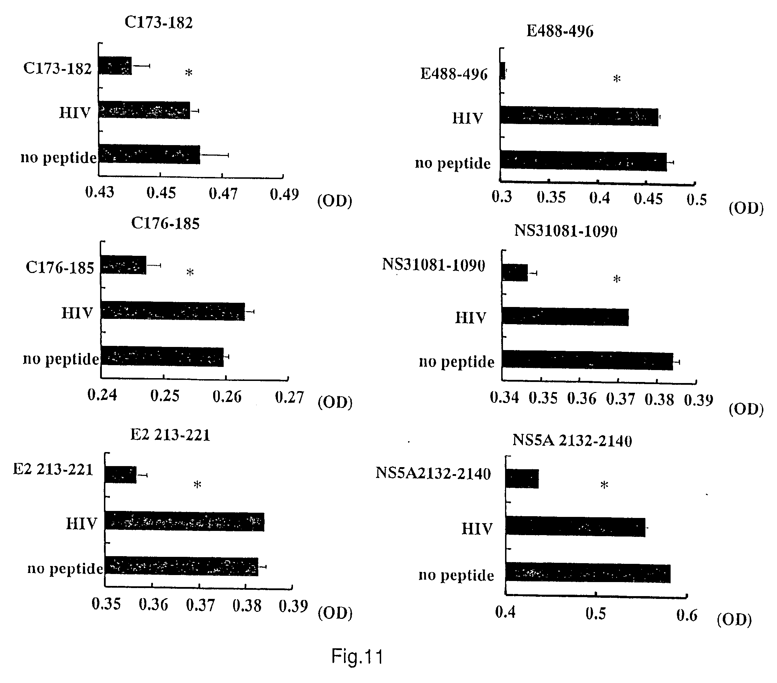

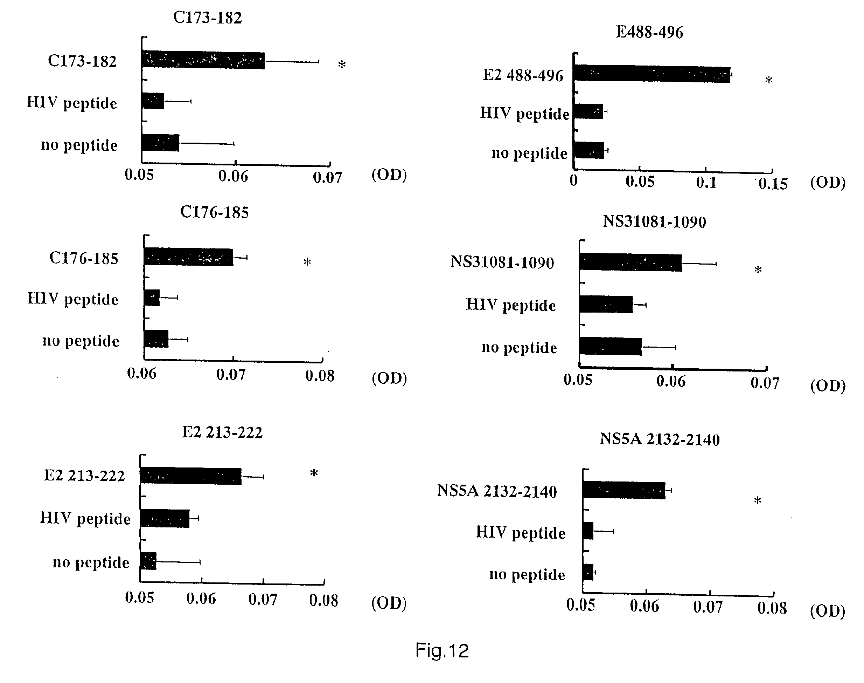

The peptide specificity of IgG reactive to each of these peptides was examined by absorption and elution tests. Each serum sample was absorbed by either a corresponding peptide or an irrelevant peptide (HIV; used as a negative control) at 37°C for 3 times and the level of peptide-specific IgG was measured by ELISA (Fig. 11). In the elution test, IgG molecules bound to a peptide- immobilized plate was eluted with either a corresponding peptide or an irrelevant peptide after the first absorption, and then the level of peptide-specific IgG in an eluted fraction was measured by ELISA. Representative results of the mean ± SD of OD values from three measurements is shown in the figure (Fig. 12). The asterisks indicate p < 0.05 in the two-sided Student's t-test.

As expected, each IgG to the 6 types of peptides was all absorbed by the corresponding peptide, but not absorbed by the irrelevant peptide (HIV peptide). Furthermore, this IgG was eluted from a binging fraction by the corresponding peptide, but not eluted by the irrelevant peptide. Taken together, these results suggested that IgG reactive to each peptide be specific to the peptide derived from HCV-1b.

### Induction of peptide-specific cellular response

PBMCs from HLA-A24⁺ patients infected with HCV-1b (n = 12, 6 CH patients, 3 LC patients, and 3 HCC patients) and HLA-A24⁺ HDs (n = 5) without HCV infection were cultured with each of the 6 types of peptides (purity > 90%) or a control HIV peptide for 13 days, and examined for IFN production in response to C1R-A2402 cell pulsed with a corresponding peptide. PBMCs from HLA-A24⁺ HCV-1b⁺ patients (n = 12) and HDs (n = 5) were stimulated with any of the 6 types of peptides in 4 wells (15 x 10⁴/well). On day 14 of the culture, the peptide-stimulated PBMCs (80 to 120 x 10⁴/well) were separately collected from each well and divided into quarters. Two portions of them were tested separately for the ability to produce IFN-γ in response to C1R-A2402 cells pulsed with the corresponding peptide. The remaining two portions were tested with cells using the negative control peptide (HIV). The level of IFN-γ produced in response to the HIV peptide (< 50 pg/ml) was subtracted as background. The asterisks indicate p < 0.05 in the two-sided Student's t-test.

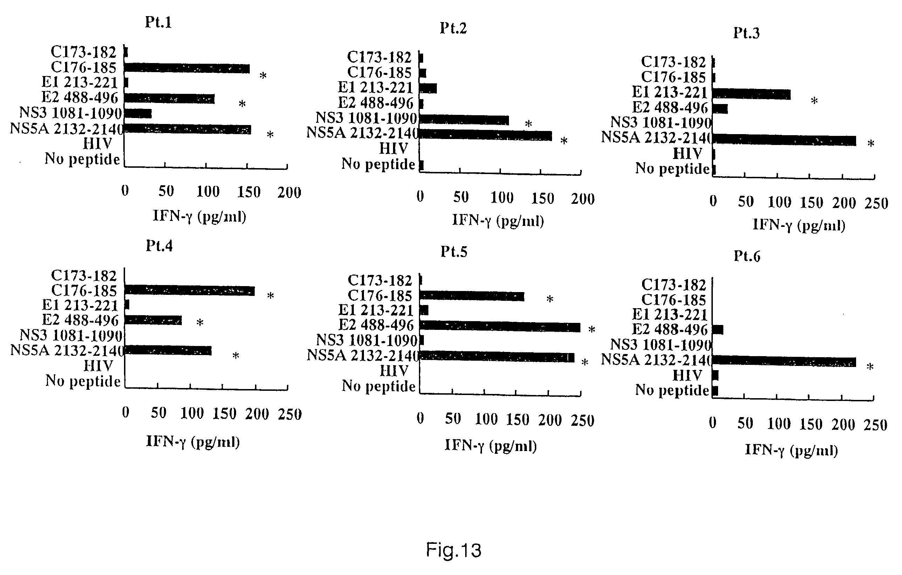

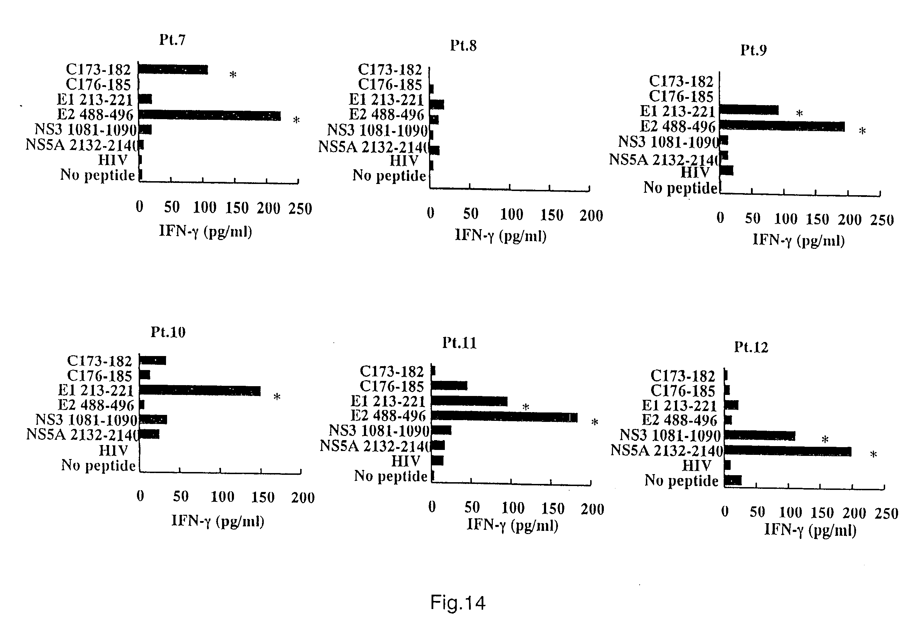

HCV peptides of C-173, C-176, E1-213, E2-488, NS3-1081 and NS5A-2132 induced IFN-γ production at a significant level (p < 0.05) in 1, 3, 4, 6, 2 and 7 of 12 patients (Figs. 13 and 14) and in 0, 0, 1, 1, 1, and 1 of 5 HDs (data not shown), respectively. Among these 6 types of peptides, NS5A-2132 peptide was recognized by cellular immunity and humoral immunity in PBMCs from 7 of 12 patients and in the sera of 57 of 60 HCV-1b⁺ patients tested.

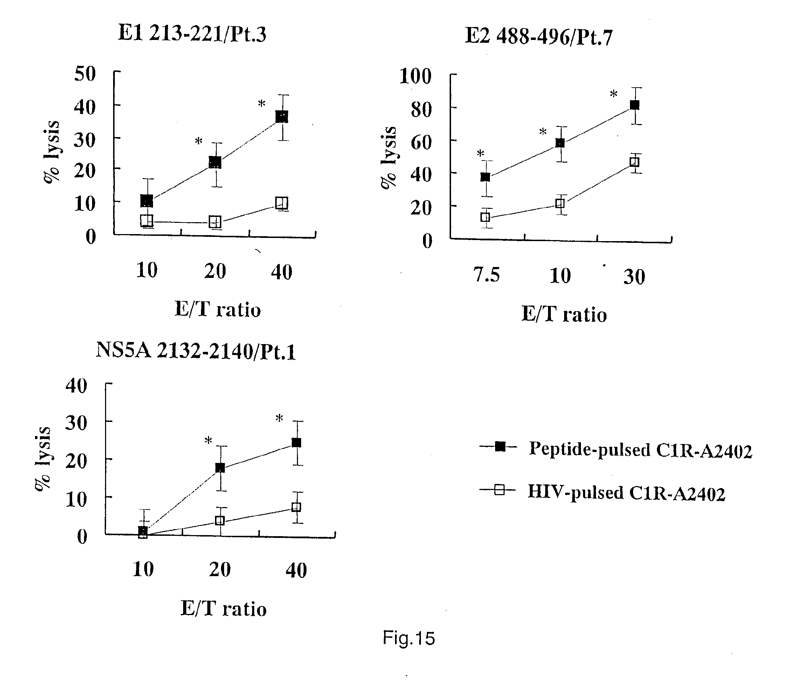

The cytotoxicity of these peptide-stimulated PBMC was examined by a 6-hour ⁵¹Cr release assay (Fig. 15). Peptide-stimulated PBMC showing positive response in the IFN-γ production assay (described above) was cultured and grown in vitro only with IL-2. Then, cytotoxicity against C1R-A2402 cell pulsed with a corresponding peptide or an HIV peptide (negative control) was tested by a standard 6-hour ⁵¹Cr release assay at three different E/T cell ratios. Representative results are shown in the figure. The values are expressed as the mean ± SD of percentage of specific lysis. The asterisks indicate p < 0.05 in the two-sided Student's t-test. The PBMC stimulated by NS5A-2132, E2-488, or E1-213 peptide showed a significant level of cytotoxicity against C1R-A2402 cell pre-loaded with a corresponding peptide but not with the HIV peptide (Fig. 15).

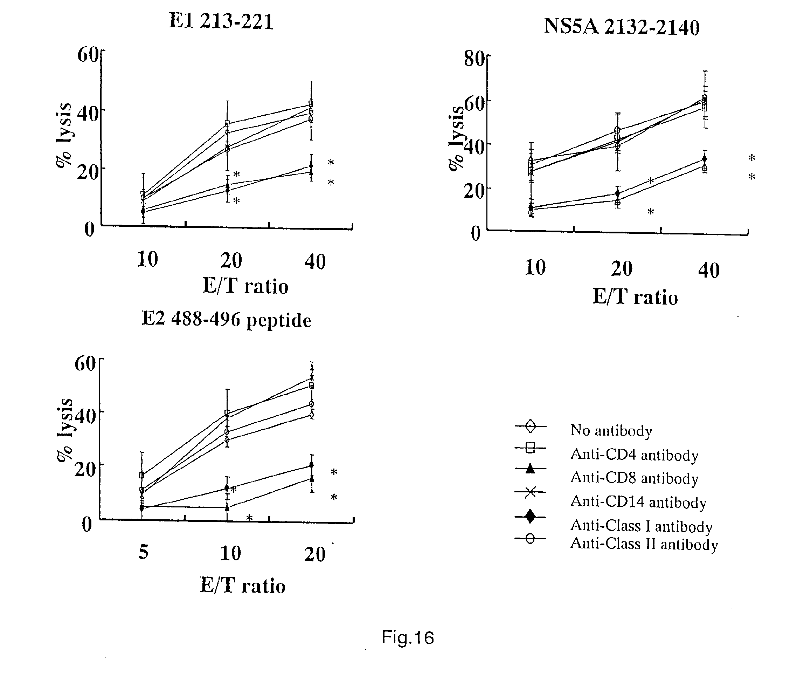

The peptide-stimulated PBMC used in the experiment shown in Fig. 15 was tested for cytotoxicity against C1R-A2402 cells pulsed with a corresponding peptide in the presence of 20 µg/ml of an anti-HLA-class I (W6/32, IgG2a), anti-HLA-class II (H-DR-1, IgG2a), anti-CD8 (Nu-Ts/c, IgG2a) or anti-CD4 (Nu-Th/i, IgGl) monoclonal antibody (mAb). An anti-CD14 (JML-H14, IgG2a) mAb was used as a negative control. A 6-hour ⁵¹Cr release assay was carried out at three different E/T ratios. The values are expressed as the mean ± SD of percentage of specific cytotoxicity (Fig. 16). The asterisks indicate P < 0.05 in the two-sided Student's t-test.

Cytotoxicity against C1R-A2402 cells pulsed with each of 3 types of peptides was inhibited by the anti-HLA-class I (W6/32) or CD8 monoclonal antibody (mAb), but not by any of the other mAbs tested, indicating that the peptide-specific cytotoxicity is mediated mainly by CD8⁺T cells in an HLA-class I restricted manner (Fig. 16). On the contrary, such cytotoxicity was not detectable in the PBMCs stimulated with the remaining 3 types of peptides (C-173, C-176 and NS3-1081) (data not shown).

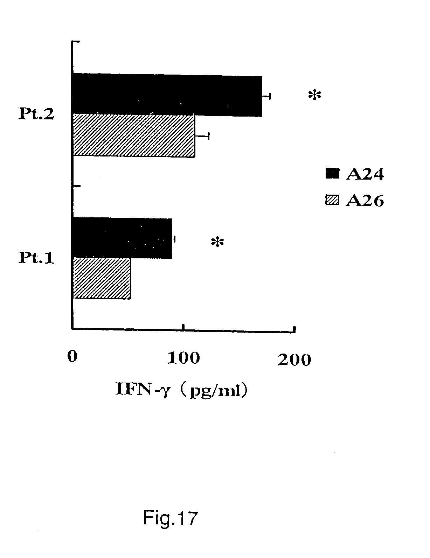

Then, it was examined whether NS5A-2132 peptide-stimulated PBMCs recognize a naturally processed peptide using COS-7 cell that was transiently co-transfected with NS5A and HLA-A2401 genes as a target cell. As a negative control, COS-7 cell that was transiently co-transfected with NS5A and HLA-A2601 genes was used. The NS5A-2132 peptide-stimulated PBMCs from patients #1 and #2 were tested for their ability to recognize COS-7 cell transiently co-transfected with NS5A and HLA-A2401 genes as a target cell to produce IFN-γ. As a negative control, COS-7 cell that was transiently co-transfected with NS5A and HLA-A2601 genes was used. The values are expressed as the mean ± SD of percentage of IFN-γ production in triplicate assays at an E/T ratio of 20:1 (Fig. 17). The asterisks indicate p < 0.05 in the two-sided Student's t-test.

As expected, the peptide-stimulated PBMCs from patients #1 and #2 showing a CTL activity (Fig. 13) recognized COS-7 cell co-transfected with NS5A and HLA-A2401 genes and produced a significant amount of IFN-γ (Fig. 17). On the contrary, these PBMCs did not react with COS-7 cell having NS5A and HLA-A2601 genes as a negative control. In addition, the peptide-stimulated PBMCs from other two patients (#3 and #4) who did not show a CTL activity failed to produce a significant amount of IFN-γ by the recognition of COS-7 cell co-transfected with NS5A and HLA-A2401 genes (data not shown).

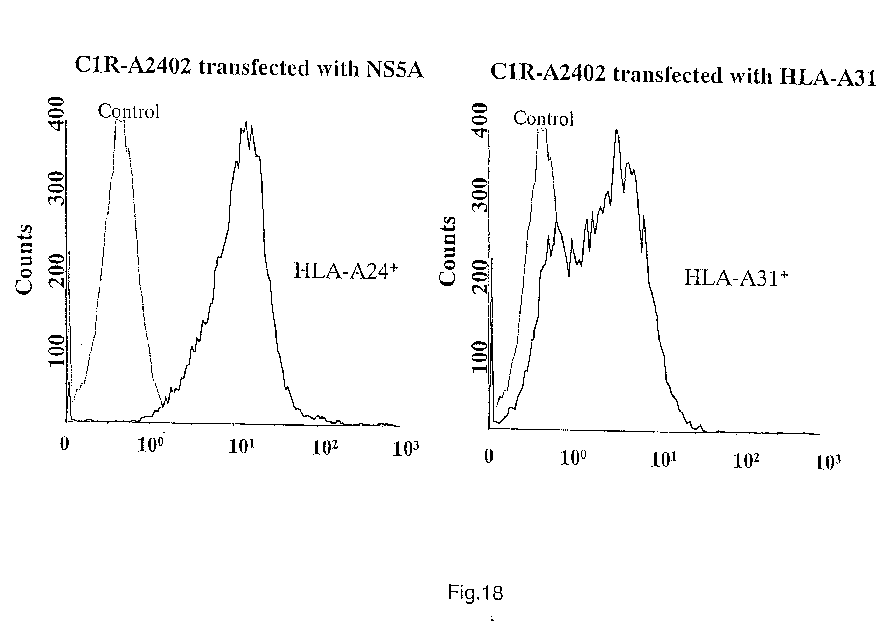

Next, cytotoxicity was tested against C1R-A2402 cell that was stably transfected with NS5A gene and used as a target cell. The expression of HLA-A24 molecule in C1R-A2402 cell that was stably transfected with NS5A gene and the expression of HLA-A31 molecule in C1R-A2402 cell that was stably transfected with HLA-A31 gene (negative control) were analyzed by a flow cytometry using a FACScan (Fig. 18).

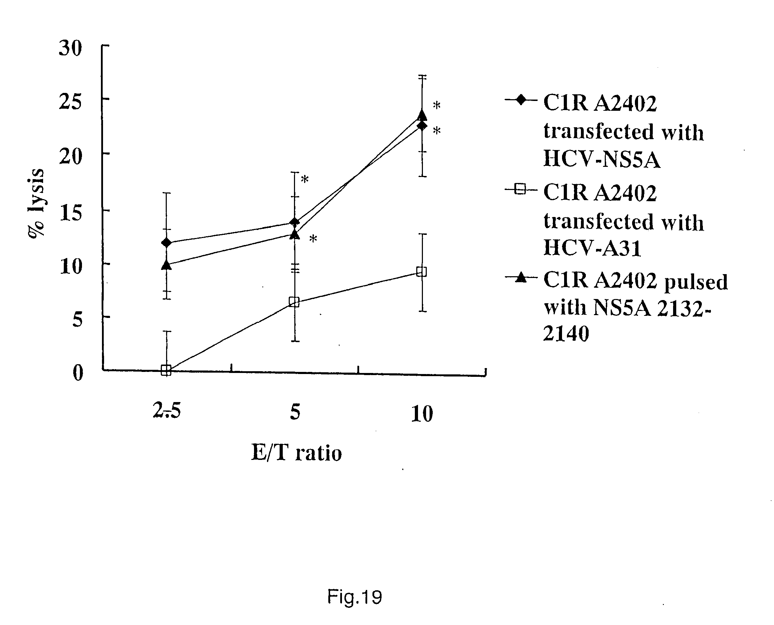

Then, cytotoxicity was tested by a 6-hour ⁵¹Cr release assay using C1R-A2402 cell that was stably transfected with NS5A gene as a target cell. C1R-A2402 cell that was stably transfected with a negative control gene (HLA-A3101) was used as a negative control, and C1R-A2402 cell that was pulsed with NS5A-2132 peptide was used as a positive control. A 6-hour ⁵¹Cr release assay was carried out at three different E/T ratios. The values are expressed as the mean ± SD of percentage of specific lysis. The asterisks indicate p < 0.05 in the two-sided Student's t-test. The PBMC stimulated with NS5A-2132 peptide showed a higher cytotoxicity level against both C1R-A2402 cell that was transfected with NS5A gene and non-transfected C1R-A2402 cell that was previously pulsed with a corresponding peptide, compared with the cytotoxicity against C1R-A2402 cell that was transfected with the negative control gene (Fig. 19). These results suggest that NS5A-2132 peptide-stimulated PBMC successfully recognized a peptide processed and produced naturally on HLA-A2402 molecule of HCV-1b⁺ cell.

### Further examination on anti-NS5A-2132 IgG

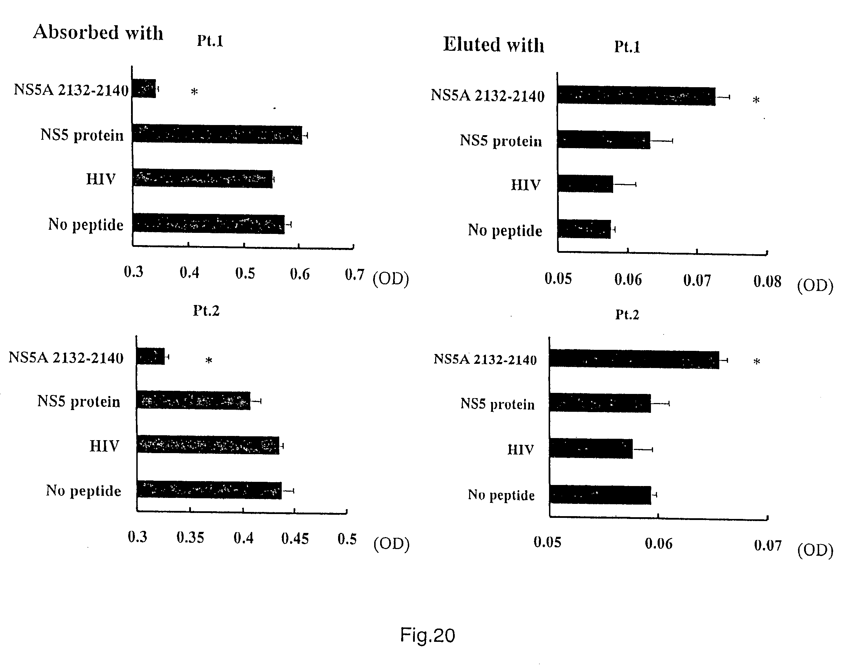

In order to understand more about the possibility of biological role of anti-peptide IgG, it was examined whether anti-NS5A-2132 IgG recognizes the whole NS5A protein in absorption and elution assays. NS5A-2132 and an HIV peptide were used as a positive control and a negative control, respectively (see the description above for the details of the absorption and elution tests). Anti-peptide IgG was neither absorbed nor eluted with the whole NS5 protein (Fig. 20), suggesting that this peptide IgG did not react with the whole NS5 protein.

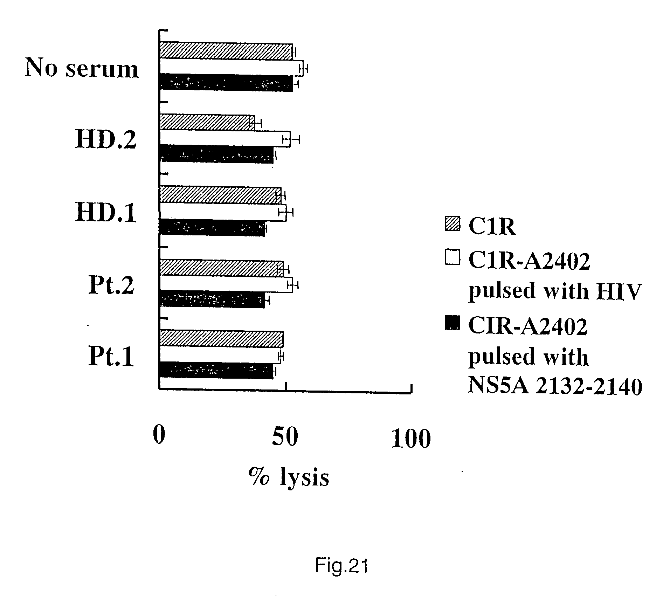

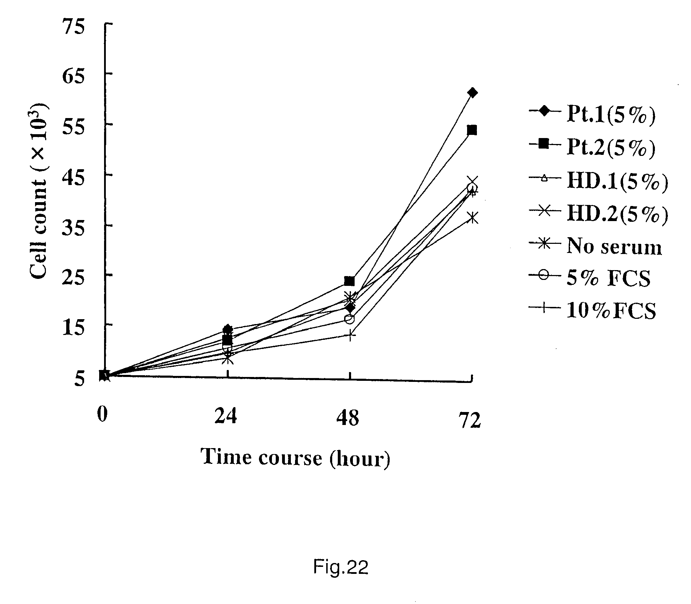

Then, Huh7 cells were incubated up to three days in the presence of sera from patients (sera from 2 HCV-1b⁺ patients whose sera showed a high level of anti-NS5A-2132 activity). As a negative control, sera from 2 HDs and FCS were used. The number of Huh7 (NNU50-1) cells that could survive was counted with a Cell Count kit-8 (10 µl/well) and the mean value of three assays are shown. None of the tested sera inhibited the growth of Huh7 (NNU50-1) cells under these culture conditions (Fig. 21). Also it was examined whether anti-NS5A-2132 IgG has the ability to mediate ADCC activity. PBMC newly isolated from an HLA-A24⁺ HD was tested for cytotoxicity against C1R-2402 cell previously pulsed with this peptide in the presence of the above-mentioned 4 types of inactivated sera. However, none of the sera tested showed ADCC activity under these conditions (Fig. 22).

### Example 3: Prediction of prognosis of patient infected with HCV

### Subjects

Sera were obtained from 33 patients with a disease associated with HCV in the cohort study conducted between 1995 and 2002. The results of the follow-up survey on the 33 patients are shown in Table 4. The sera obtained from patients with chronic hepatitis (CH, n = 68), liver cirrhosis (LC, n = 43) and hepatic cell carcinoma (HCC, n = 52) were also used in the analysis. These patients were diagnosed at the time of the first serum sampling by biochemical and histological findings, echography and computed tomography. An anti-HCV antibody was assayed using a chemiluminescence enzyme immunoassay (CLEIA) kit (Lumipulse II HCV, Fujirebio Inc., Tokyo, Japan) or the second generation or the third generation enzyme immunoassay test (SRL, Tokyo, Japan). HCV-RNA in the sera was detected using RT-PCR (SRL, Tokyo, Japan). The HCV genotype was determined by directly sequencing HCV in the sera of the patients by RT-PCR (SRL, Tokyo, Japan). The sera were also obtained from subjects who were not infected with HCV, which include 24 subjects with an autoimmune disease (6 patients with systematic lupus erythematosus, one with Behcet's disease and 17 with atopic dermatitis), 17 cases of hepatitis B virus (HBV) infection (positive for HBV surface antigen), 3 patients having human immunodeficiency virus (HIV) and 10 cases with human T cell leukemia virus type I (HTLV-1) infection. As a negative control, the sera obtained from 37 persons who did not have the history of a viral hepatitis or vaccination of HBV and had normal hepatic function were tested.

**Table 4: Diagnosis results in 1995 and 2002**

| 1995 | n | 2002 | n | Sex (M/F) | Age ± SD |
|---|---|---|---|---|---|
| CH | 17 | CH | 12 | 3/9 | 67.7 ± 11.9 |
| | | LC | 3 | 2/1 | 59.0 ± 7.9 |
| | | CH; with HCC | 1 | -/1 | 82 |
| | | LC; with HCC | 1 | 1/- | 65 |
| LC | 1 | LC | 1 | -/1 | 69 |
| ASC | 4 | CH | 1 | -/1 | 75 |
| | | ASC | 1 | -/1 | 76 |
| | | Past medical history of HCV infection | 2 | -/2 | 57.7 ± 6.36 |
| Past medical history of HCV infection | 11 | Past medical history of HCV infection | 11 | 2/9 | 68.8 ± 10.71 |
| Total | 33 | | 33 | 8/25 | 67.1 ± 10.6 |

| | | | | | |
|---|---|---|---|---|---|
| CH: chromic hepatitis; LC: liver cirrhosis; HCC: hepatic cell carcinoma; ASC: asymptomatic healthy carrier | | | | | |

### Peptide

Two peptides with a purity of 90% or more were purchased from BIOSYNTHESIS (Lewisville, TX): HCV-1b core protein 35-44 (YLLPRRGPRL (SEQ ID NO: 1) capable of inducing an HLA-A2-restricted CTL activity) and HCV-1b NS5A protein 2132-2140 (RYAPACKPL (SEQ ID NO: 2) capable of inducing an HLA-A24-restricted CTL activity). As a negative control, peptides derived from HIV with an HLA-A2-binding motif (SLYNTVATL (SEQ ID NO: 64)) and an HLA-A24-binding motif (RYLRDQQLLGI (SEQ ID NO: 63)) were used. Assay of antibody reactive to peptide

The level of peptide-specific IgG was measured by an enzyme-linked immunosorbent assay (ELISA). Briefly, each peptide was dissolved in dimethyl sulfoxide (DMSO) and stored at -20°C. The peptide (20 µg/well) diluted with 0.1 M carbonate buffer containing disuccinimidyl suberate (DSS) (PIERCE, Rockford, IL) as a chemical crosslinker was bound to an ELISA plate. The wells were washed 3 times with 0.05% Tween 20/PBS (PBST). Then, the plate was blocked overnight at 4°C with Block Ace (Yukijirushi, Tokyo, Japan). A serum sample was diluted 1:100, 1:200 or 1:400 with 0.05% Tween 20/Block Ace, and 100 µl/well of the sample was added to each well. After the sample was incubated at 37°C for 2 hours, the plate was washed 9 times with PBST, and further incubated at 37°C for 2 hours with 100 µl/well of a 1:1000 diluted rabbit anti-human IgG (γ-chain specific) (DAKO Glostrup, Denmark). After the plate was washed 9 times, 100 µl/well of a 1:100 diluted anti-rabbit IgG-conjugated horseradish peroxidase- dextran polymer (En Vision, DAKO) was added to each well, and then the plate was incubated at room temperature for 40 minutes. After the plate was washed, 100 µl/well of a tetramethylbenzidine substrate solution (KPL, Guildford, UK) was added, and the reaction was stopped by the addition of 1.0 M phosphoric acid.

### Statistics

A statistical analysis was carried out using the χ²-test. Values of P < 0.05 were considered statistically significant.

### Cross reactivity, HLA restriction and genotype restriction

The cross reactivity of two antibodies reactive to the peptides derived from HCV-1b was examined. The sera obtained from 60 HCV patients and patients who are not included in the cohort study (CH, n = 22, LC, n = 21 and HCC, n = 17) were examined for reactivity to the peptides of the core 35-44 (C-35) and NS5A 2132-2140 (NS5A-2132). The sera were also obtained from 24 subjects with an autoimmune disease, 17 cases of HBV infection and 10 cases of HTLV-1 infection. The sera obtained from 37 HDs were used as a negative control. The level of peptide-reactive IgG contained in the serially diluted serum samples was measured by ELISA. The results are reported by the absorbance (OD) of each sample. The representative results of OD at a serum dilution of 100:1 are shown. The cut-off value was set at 0.093 (mean + 2 SD of OD from HDs). The statistical analysis was carried out using the χ²-test. Values of P < 0.05 were considered statistically significant.

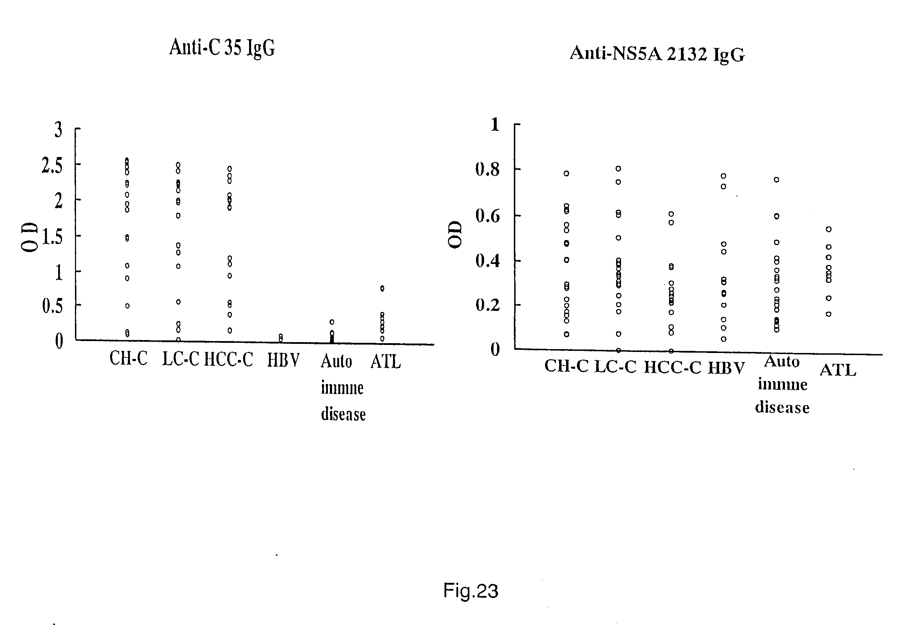

As a result, a significant level of anti-C-35 IgG was detected in 56 out of 60 HCV-positive patients (93.3%) and there was no significant difference in the IgG level among the 3 groups (CH, LC and HCC patients) (Fig. 23). Except for the HTLV-1 patients, the sera from different groups were not positive for the anti-C-35 antibody. In the sera of 8 out of 10 cases of HTLV-L⁺ subjects, a low but significant level of anti-C-35 IgG was detected. A significant level of anti-NS5A-2132 IgG was detected in 45 out of 60 patients (75%). The IgG level was high in the CH patients, intermediate in the LC patients and low in the HCC patients in the 3 groups (p < 0.05 vs CH) (Fig. 23). The sera from other groups were positive for anti-NS5A-2132 IgG in the majority of tested cases. Further, the sera of 3 out of 37 HDs were positive for anti-NS5A-2132 IgG.

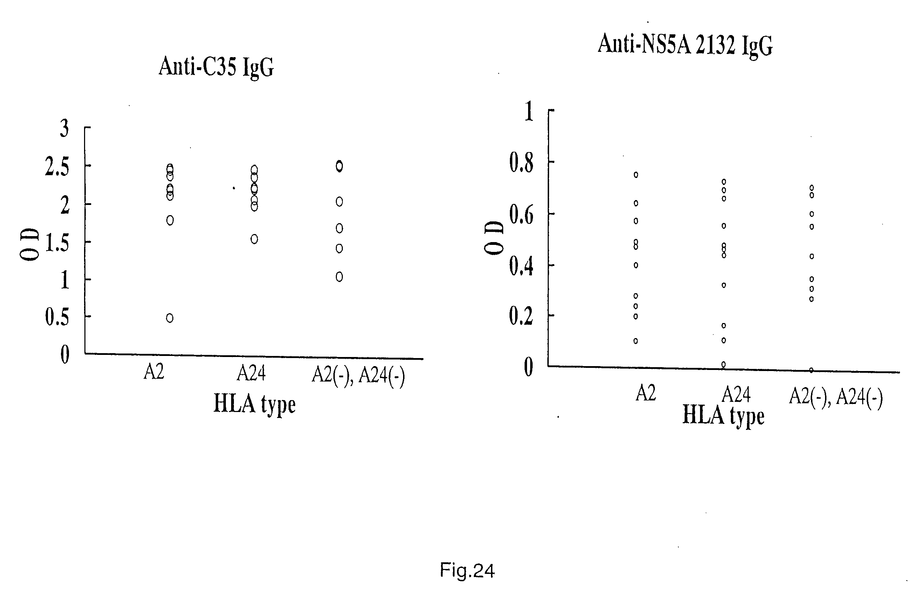

Then, the correlation between the HLA-class IA phenotype and the level of anti-C-35 IgG or anti-NS5A-2132 IgG was examined in 29 patients with a disease associated with HCV. The HLA-class IA phenotype was determined by a standard serological method. Nine patients were HLA-A2⁺, 13 patients were A24⁺, and the remaining 7 patients were A2⁻A24⁻. The levels of Anti-C-35 and anti-NS5A-2132 were measured by a standard ELISA and the OD value of each patient at a serum dilution of 100:1 is shown in the figure. Regardless of difference in the HLA-class IA phenotype, both antibodies were detected in the majority of the patients infected with HCV (Fig. 24).

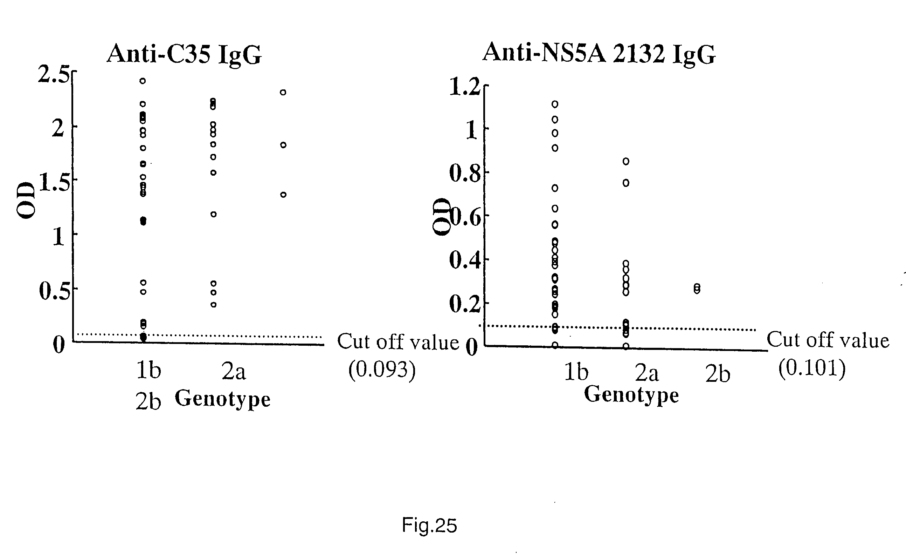

Also the correlation between the HCV genotype and the level of anti-C-35 IgG or anti-NS5A-2132 IgG was examined in a double blind study in patients infected with HCV-1b (n = 29), HCV-2a (n = 16) and HCV-2b (n = 3). The HCV genotype was determined by directly sequencing HCV in the serum of the patient. The results of all subjects at a dilution of 100:1 are shown in Fig. 25. The levels of anti-C-35 and anti-NS5A-2132 were measured by a standard ELISA and the OD value of each patient at a serum dilution of 100:1 is shown. The cut-off value was set at 0.093 (mean + 2 SD of OD from HDs).

The anti-C-35 antibody was detected in 26 out of 30 patients infected with HCV-1b, 15 out of 15 patients infected with HCV-2a and 3 out of 3 patients infected with HCV-2b, respectively. Similarly, the anti-NS5A-2132 antibody was detected in the sera of 23 out of 30 patients infected with HCV-1b, 10 out of 16 patients infected with HCV-2a and 3 out of 3 patients infected with HCV-2b, respectively (Fig. 25). These results indicate that both anti-C-35 antibody and anti-NS5A-2132 antibody were detected in the HCV-positive patients regardless of difference in the HLA-class IA subtype and in the HCV genotype.

The above results suggest that the level of the anti-NS5A-2132 antibody correlates with the prognosis of an individual infected with HCV but not the case with the anti-C-35 antibody.

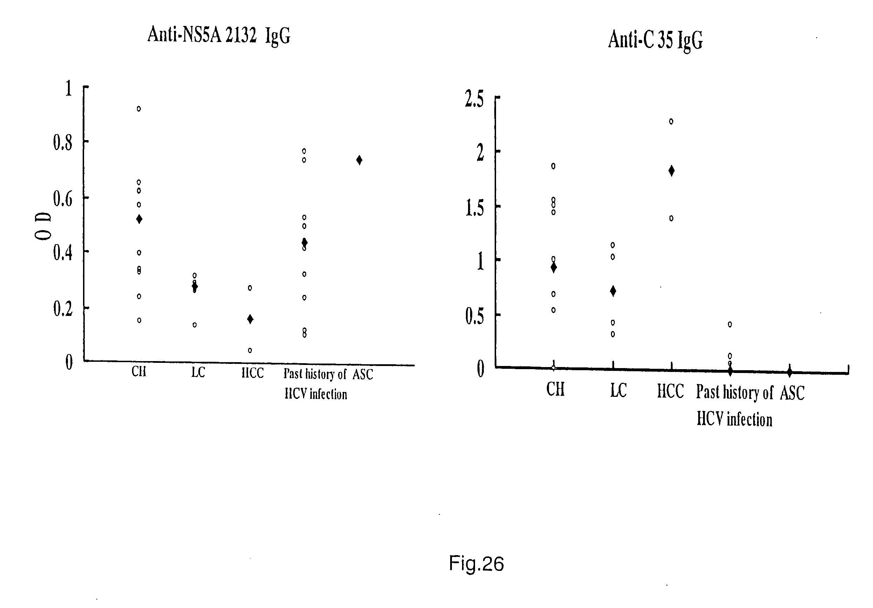

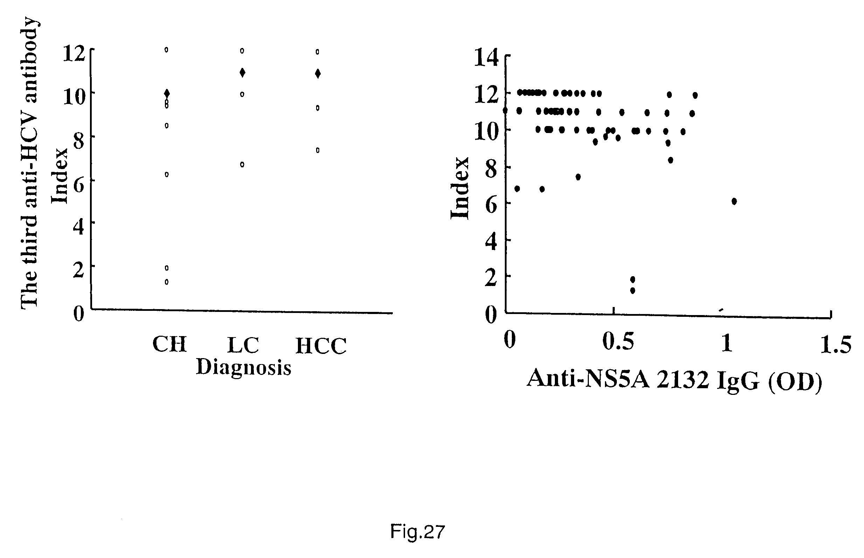

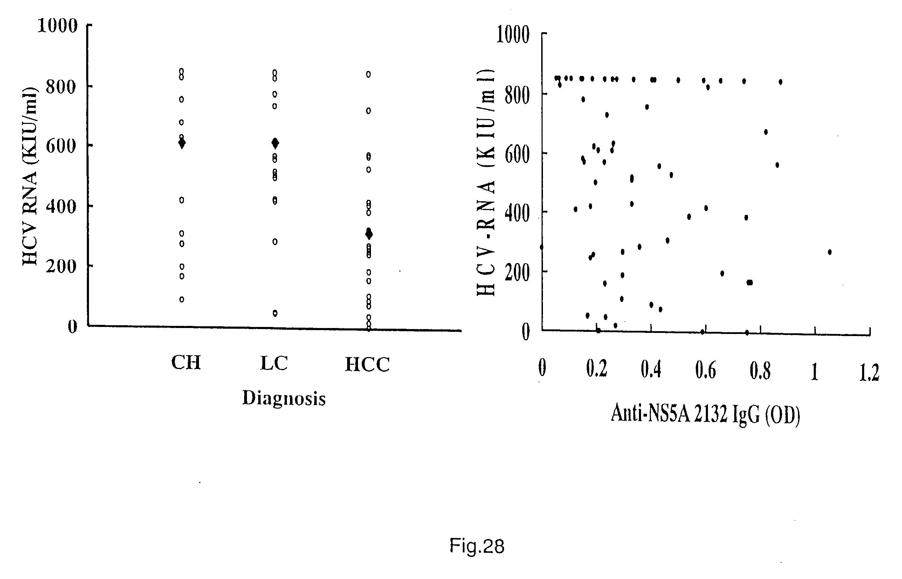

Then, the sera of CH (n = 24), LC (n = 22), HCC (n = 26) and HDs (n = 9) were newly prepared and the level of anti-NS5A-2132 IgG was measured (Fig. 26). The values of mean ± SD for the CH, LC, HCC and HD groups were 0.51 ± 0.24, 0.27 ± 0.20, 0.26 ± 0.23 and 0.08 ± 0.07, respectively. The levels of the anti-NS5A-2132 antibody in the LC and HCC patients were significantly lower than that in the CH patients (p < 0.05), but still higher than that in the HDs. In order to compare these results with the results measured by a standard third generation assay, the level of anti-HCV antibody was measured for all the sera using a commercially available kit (Third generation assay, SRL). The level is represented by an exponent (the left column). The correlation between the anti-HCV level and the anti-NS5A- 2132 level represented by an exponent is shown in the right column. The levels of the anti-HCV antibody measured using the third generation assay were not significantly different among the 3 groups (CH: 10 ± 2.7, LC: 11 ± 1.4, HCC: 11 ± 1.1) (Fig. 27). Further, the HCV RNA level was measured for these sera using a commercially available kit (SRL, Japan). The HCV RNA level of the HCC patients (360 ± 269.6) was lower than that of the CH patients (610 ± 347.3) or the LC patients (610 ± 246.4) (p < 0.05) (Fig. 28). However, there was no apparent correlation between the level of the anti-NS5A antibody and the HCV RNA level (Fig. 28).

### Result of cohort study

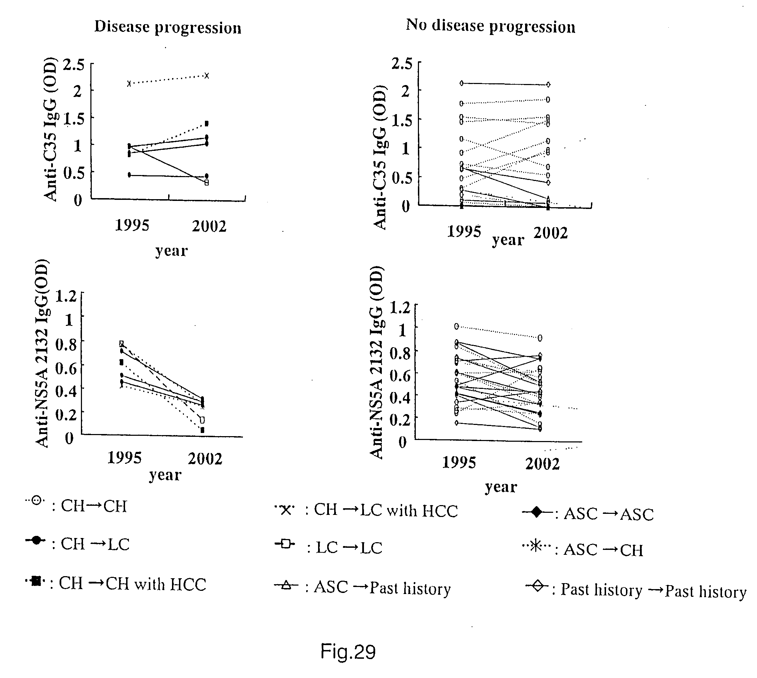

In order to further examine the correlation between the anti-NS5A-2132 antibody and the prognosis of an HCV-positive patient at an individual level, the serum levels of the two antibodies were examined in a sample from the cohort study, in which habitants infected with HCV were annually screened from 1990 to 2002. This study was done with 66 serum samples in total, which were obtained from 33 patients and collected twice in 1995 and 2002 from the same individuals. The diagnoses of these 33 patients were CH (n = 17), LC (n = 1), asymptomatic carrier (ASC) (n = 4), and a past medical history of HCV infection (an individual spontaneously recovered) (n = 11) at the time of 1995, and CH (n = 13). LC (n = 4), HCC (n = 2), asymptomatic carrier (ASC) (n = 1), and a past medical history of HCV infection (n = 13) at the time of 2002. In other words, in the period of seven years, the progress of disease was not observed in 26 patients, while the progress was observed in the remaining 7 patients (Table 4). The OD values of the anti-C-35 antibody and the anti-NS5A-2132 IgG in each patient measured at a serum dilution of 100:1 in 1995 and 2002 are shown in Fig. 29. As expected, the levels of the anti-C-35 antibody measured in 1995 and 2002 were almost equal regardless of the conditions of the disease in the majority of the 33 cases. On the contrary, the level of the anti-NS5A antibody measured in 1995 decreased when measured in 2002 in all the 7 patients whose diseases had progressed, whereas the values were almost equal to those measured in 2002 in the majority of the 25 cases in which the diseases had not progressed. The median value ± SD of the anti-NS5A-2132 antibody in the sera of the 7 patients whose diseases had progressed measured at the time of 1995 (0.67 ± 0.13) was significantly higher than the value (0.27 ± 0.11) measured in 2002 (p < 0.05).

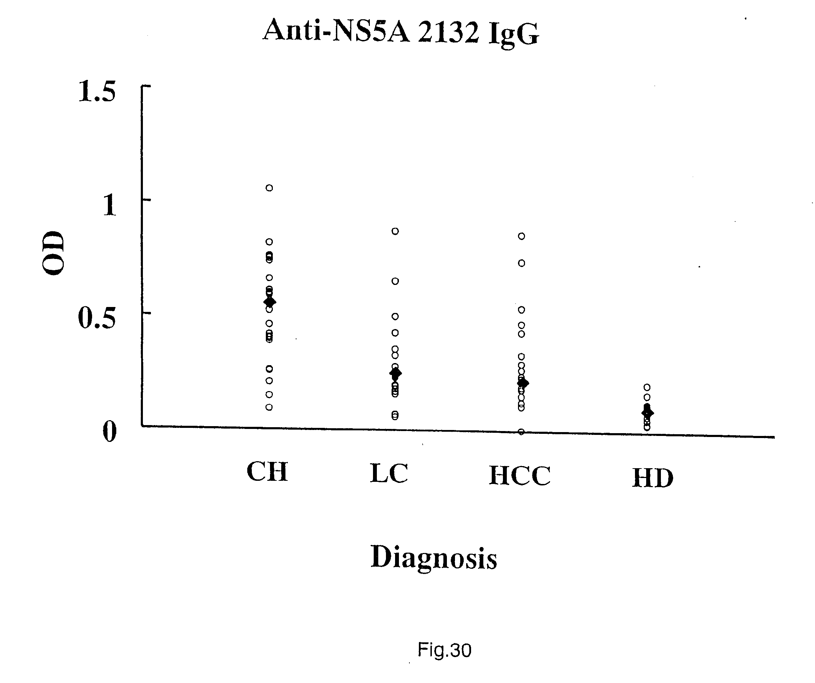

Then, the 33 subjects were subdivided into 5 groups (CH, LC, HCC, a past medical history of HCV infection (a recovered individual), and ASC) and the levels of the two antibodies at a serum dilution of 100:1 in 2002 were plotted (Fig. 30). A statistical analysis was carried out using the χ²-test. Values of P < 0.05 were considered to be statistically significant. The level of the anti-C-35 antibody was high in any of CH, LC, and HCC patients, and it became very low or undetectable in the recovered individuals. On the other hand, the level of the anti-NS5A-2132 antibody was high in CH, the recovered individual and ASC, intermediate in LC and the lowest in the HCC patients (p < 0.05 vs CH).

### Example 4: Detection of IgG reactive to HCV peptide in the sera of HLA-A24⁺ HCV-infected patients using Luminex assay

The level of IgG reactive to HCV peptide in the sera of HLA-A24⁺ HCV-infected patients was measured in the same manner as in Example 1 using the Luminex assay, which is considered to be more sensitive than the ELISA method. Coupling of peptide to microbeads

Each peptide was coupled to microbeads (manufactured by Luminex Corporation, xMAP Multi-Analyte COOH Beads), which was encoded with a classification code for the content of each fluorescent dye (hereinafter referred to as color-coded) according to the manufacturer's instruction. One hundred microliters of unprepared color-coded microbeads were put in each well of a filter plate and aspirated, and then washed twice with a washing buffer (phosphate buffered saline (PBS) (pH 7.4 ± 0.1), Tween (registered trademark) 20 (0.05% v/v)) and each well was aspirated at the same time. Then, 50 µl of 0.1 M MES (2-morpholinoethanesulfonic acid) buffer (pH 7.0) was added, and 10 µl of EDC (N-ethyl-N'-(3-dimethylaminopropyl)-carbodiimide hydrochloride) (1 mg/ml/0.1 M MES buffer (pH 7.0)) was added to each well. Several hundred microliters of the peptide (1 mg/ml, 0.1 M MES buffer, pH 7.0) was mixed with the washed microbeads in each well. After the peptide and the mixed microbeads were reacted in the dark for 20 minutes at room temperature, 10 µl of EDC (1 mg/ml/0.1 M MES buffer (pH 7.0)) was added to each well and the mixture was allowed to react in the dark for 20 minutes at room temperature. this process was repeated twice. After the excess solution was aspirated, 100 µl of 1 M Tris-HCl buffer (pH 7.0) was added to each well and the mixture was allowed to react in the dark for 15 minutes at room temperature. Then, the beads in each well were washed 3 times with the washing buffer as above and collected in a storage solution containing 0.05% sodium azide in Block Ace (registered trademark).

### Preparation of microbead mixture

Microbeads for each well are prepared by placing a bead solution obtained by binding a peptide to the color-coded microbeads in such a manner that about 5,000 microbeads are contained in each well of a filter plate (each well contains about 1 µl of single type of bead). A bead mixture was prepared by mixing equal amount of ten types of microbeads prepared in this manner and diluting the mixture with the above-mentioned washing buffer (PBS, TWEEN (registered trademark) 20 (0.05% v/v)) so as to give a total amount of about 25 µl.

### Preparation of sample

Serum was used in this study. One hundred microliters of each serum dilution was prepared by diluting the serum at 1:100 to 1:1000 with a reaction buffer (PBS (pH 7.4 ± 0.1), Tween (registered trademark) 20 (0.05% v/v), and bovine serum albumin (BSA) 10 mg/ml).

### Assay of anti-peptide antibody

A biotinylated goat anti-human IgG (γ-chain specific) diluted with the reaction buffer was used as a secondary antibody. Streptavidin (1 mg/ml) labeled with PE (phycoerythrin) (SRPE) was diluted to 20 µl (1/50 dilution) with the above-mentioned reaction buffer and used as a fluorescent dye-labeled streptavidin.

One hundred microliters of the washing buffer was put in each well of a filter plate and then removed by aspiration. This washing step was carried out twice. Then, a 96-well filter plate containing 25 µl of the above-mentioned bead mixture in each well was washed with the washing buffer twice. After the washing, 100 µl of the sample was added to all wells. The filter plate was covered and shaken by a plate shaker (300 rpm) in the dark for 2 hours at room temperature and aspirated. Then, 100 µl of the washing buffer was put in each well and removed by aspiration. This washing step was carried out three times.

Then, 100 µl of the biotinylated secondary antibody, i.e., biotinylated goat anti-human IgG (γ-chain specific), was added to each well and the filter plate was covered and shaken by a plate shaker (300 rpm) in the dark for 1 hour at room temperature. After aspiration, 100 µl of the washing buffer was put in each well and removed by aspiration. This washing step was carried out three times.

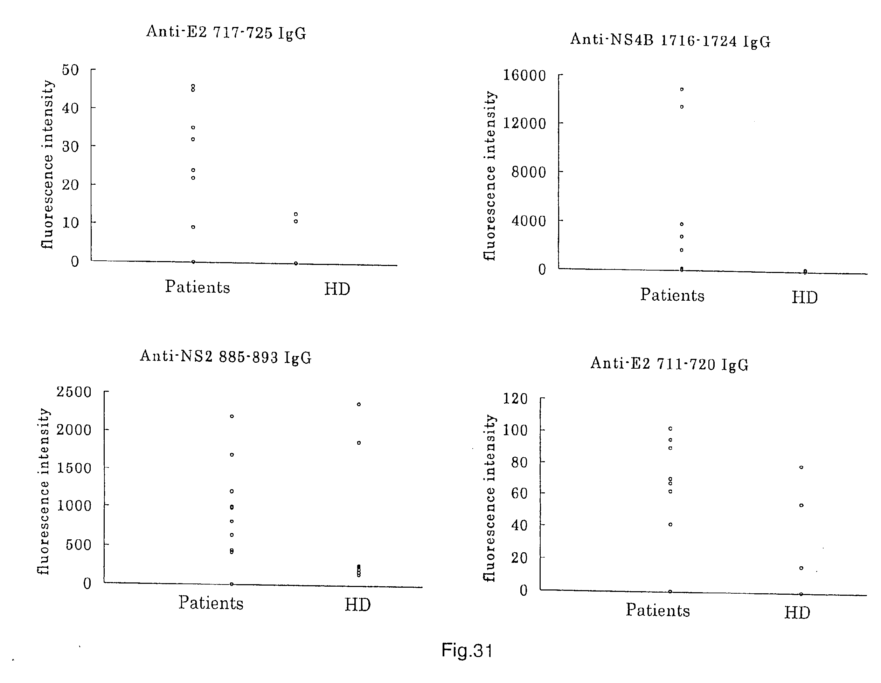

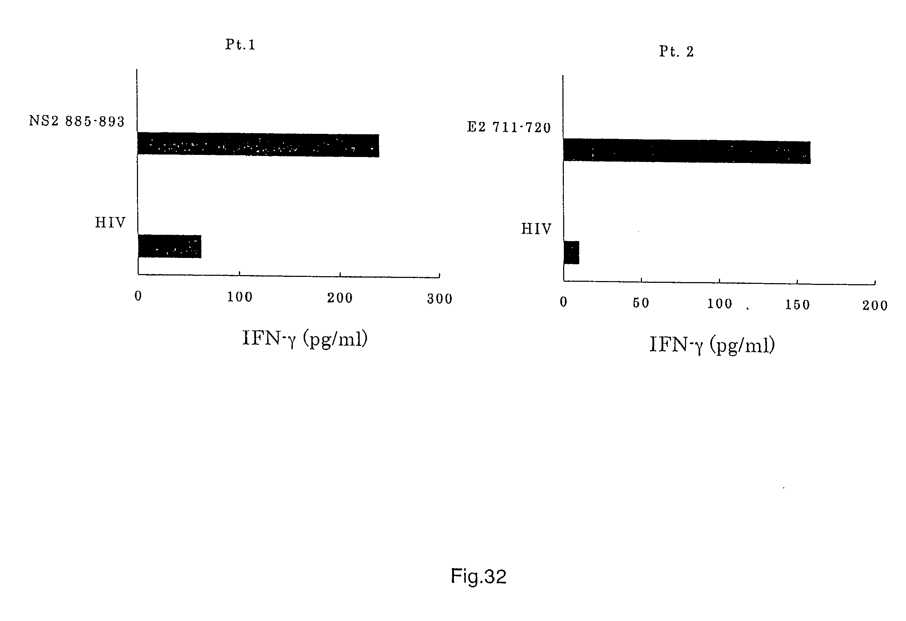

Subsequently, 100 µl of SRPE was added to each well and the filter plate was covered and shaken by a plate shaker (300 rpm) in the dark for 30 minutes at room temperature. After aspiration, 100 µl of the washing buffer was put in each well and removed by aspiration. This washing step was carried out three times. Thereafter, 100 µl of the washing buffer was added to each well and shaken by a plate shaker (300 rpm) for 2 to 3 minutes, and then 50 µl of the sample thus obtained was assayed by a fluorescence flowmetric system. The results are shown in Figs. 31 and 32 and Table 5.

**Table 5**

| No | Peptide name | Alias | Region | Sequence | SEQ ID NO | HCV patients (n = 10) | | Healthy donors (n = 10) | |
|---|---|---|---|---|---|---|---|---|---|
| | | | | | | Positive | | Positive | |
| | | | | | | n | % | n | % |
| 4 | 1bA24-717 | E2-717 | E2 | EYVLLLFLL | 8 | 6 | 60 | 2 | 20 |
| 14 | 1bA24-1716 | NS4B-1716 | NS4B | PYIEQGMQL | 16 | 6 | 60 | 1 | 10 |
| 18 | 1bA24-885 | NS2-885 | NS2 | IFTITKILL | 20 | 9 | 90 | 2 | 20 |
| 39 | 1bA24-711 | E2-711 | E2 | SFAIKWEYVL | 38 | 6 | 60 | 2 | 20 |

### Example 5: Treatment of HCV infection using peptide vaccine derived from hepatitis C virus

An anti-virus effect of a vaccine comprising a peptide derived from hepatitis C virus of the present invention was examined in HCV-infected HLA-A2⁺ or HLA-A24⁺ subjects. All the subjects were infected with HCV-1b and showed no response to a treatment with interferon and ribavirin. Peptides derived from hepatitis C virus, C-35, NS5A-2132, E2-488, E1-213 and NS3-1081 were synthesized, purified and stored as lyophilized powder under GMP compliance. The C-35, NS5A-2132, E2-488, and NS3-1081 peptides were dissolved in a small amount of DMSO (1 mg/10 to 25 µl) and E1-213 was dissolved in 7% sodium hydrogen carbonate solution for injection (Meylon) (1 mg/15 µl). Each solution was diluted with physiological saline for injection (1 to 2 mg/ml) and sterilized by filtration through 0.22 µm filter. Each of the resulting solutions was mixed with an equal amount of an adjuvant (Montanide ISA-51), to prepare emulsified injection solutions. C-35 injection solution was administered to 3 subjects of HLA-A2⁺ and NS5A-2132 injection solution was administered to 5 subjects of HLA-A24⁺. The peptide was administered every two weeks by injecting the emulsion containing 0.3 mg of the peptide per dose into the lateral region. The level of HCV RNA and the values of GOT, GPT, γ-GTP and AFP were monitored continuously.

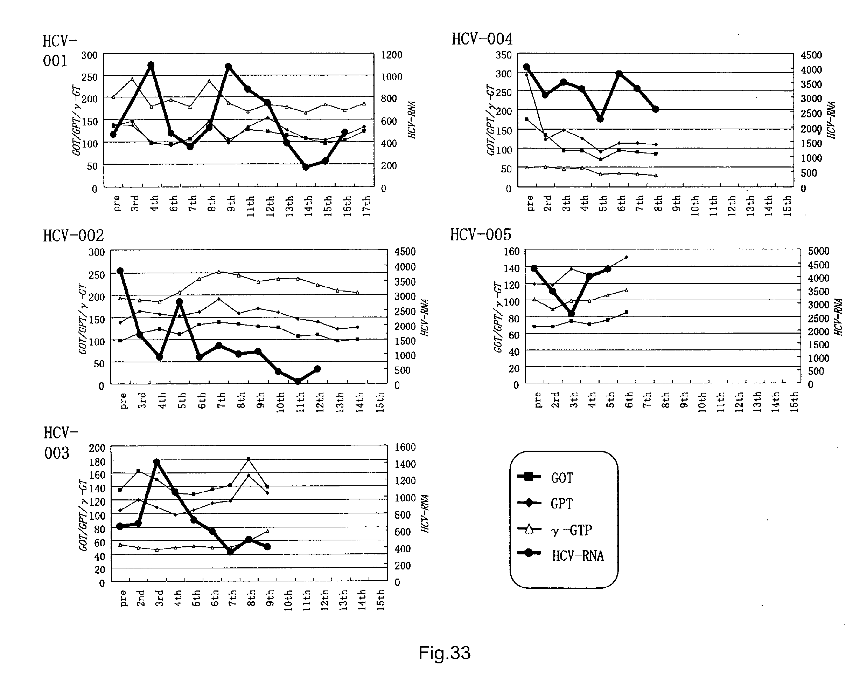

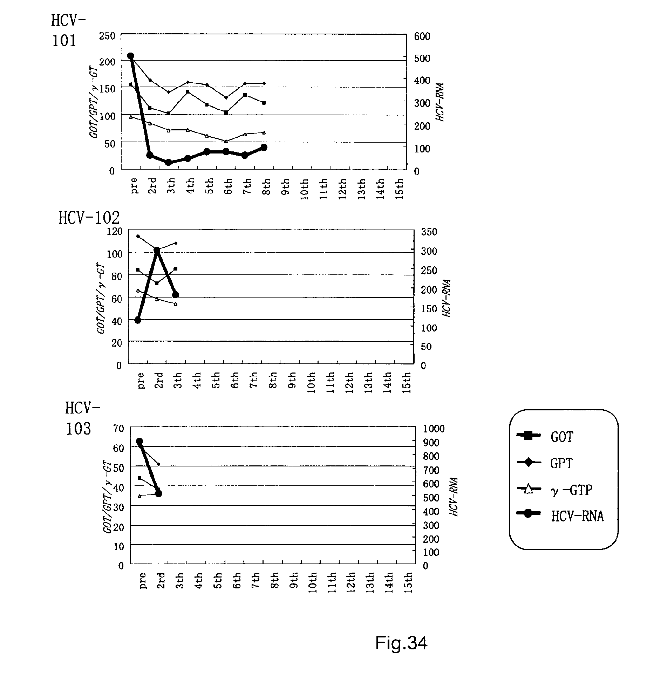

The results are shown in Fig. 33 and Fig. 34. As obvious from these drawings, the level of HCV RNA was notably decreased after the vaccine administration was initiated in the majority of the subjects assayed, demonstrating that the peptides of the present invention are effective as an anti-HCV vaccine.

### Industrial Applicability

The peptide derived from hepatitis C virus according to the invention contains an HLA-binding motif in its sequence. it is recognized by an antibody reactive to hepatitis C virus, and also has a property of being recognized by an HLA-A2- or HLA-A24-restricted cytotoxic T cell.

Accordingly, the peptide derived from hepatitis C virus of the invention could be an effective vaccine for a disease attributable to HCV infection.

The present invention furthermore relates to the following items:
1. A peptide derived from hepatitis C virus comprising an HLA-binding motif in its sequence and being recognized by an antibody detected in a patient with hepatitis C virus infection.
2. The peptide derived from hepatitis C virus according to item 1, wherein the peptide has an amino acid sequence represented by any one of SEQ ID NOS: 1 to 8, 16, 20 and 38.
3. The peptide derived from hepatitis C virus according to item 1 or 2, wherein the peptide has an amino acid sequence having a homology of at least 70% with the amino acid sequence represented by any one of SEQ ID NOS: 1 to 8, 16, 20 and 38.
4. The peptide derived from hepatitis C virus according to any one of items 1 to 3, wherein the peptide further has a property of being recognized by an HLA-A2-or HLA-A24-restricted cytotoxic T cell.
5. A polypeptide comprising a peptide derived from hepatitis C virus according to any one of items 1 to 4.
6. A polypeptide having an amino acid sequence having a homology of at least 70% with the amino acid sequence of the polypeptide according to item 5.
7. The polypeptide according to item 5 or 6, further having a property of being recognized by an HLA-A2- or HLA-A24-restricted cytotoxic T cell.
8. A nucleotide encoding a peptide derived from hepatitis C virus according to any one of items 1 to 4 or a polypeptide according to any one of items 5 to 7, or a nucleotide having a sequence complementary thereto.
9. An antibody or a substance with an antibody-like activity which recognizes a peptide derived from hepatitis C virus according to any one of items 1 to 4 or a polypeptide according to any one of items 5 to 7.
10. A vector comprising the nucleotide according to item 8.
11. A method of inducing a cytotoxic T cell by using a peptide derived from hepatitis C virus according to any one of items 1 to 4 or a polypeptide according to any one of items 5 to 7.
12. A method of detecting a hepatitis virus by using a peptide derived from hepatitis C virus according to any one of items 1 to 4, a polypeptide according to any one of items 5 to 7, a nucleotide according to item 8 or an antibody or a substance with an antibody-like activity according to item 9.
13. A method of diagnosing hepatitis C virus infection by using a peptide derived from hepatitis C virus according to any one of items 1 to 4, a polypeptide according to any one of items 5 to 7, a nucleotide according to item 8 or an antibody or a substance with an antibody-like activity according to item 9.
14. A method of preventing or treating hepatitis C virus infection by using a peptide derived from hepatitis C virus according to any one of items 1 to 4, a polypeptide according to any one of items 5 to 7, a nucleotide according to item 8 or an antibody or a substance with an antibody-like activity according to item 9.
15. A pharmaceutical composition comprising as an active ingredient a peptide derived from hepatitis C virus according to any one of items 1 to 4, a polypeptide according to any one of items 5 to 7, a nucleotide according to item 8 or an antibody or a substance with an antibody-like activity according to item 9.
16. The pharmaceutical composition according to item 15, which is a hepatitis C virus vaccine.
17. A method of predicting the prognosis of hepatitis C virus infection by using a peptide derived from hepatitis C virus according to any one of items 1 to 4, a polypeptide according to any one of items 5 to 7, a nucleotide according to item 8 or an antibody or a substance with an antibody-like activity according to item 9.
18. A kit for diagnosing hepatitis C virus infection or predicting the prognosis of hepatitis C virus infection comprising a peptide derived from hepatitis C virus according to any one of items 1 to 4, a polypeptide according to any one of items 5 to 7, a nucleotide according to item 8 or an antibody or a substance with an antibody-like activity according to item 9.

## Claims

1. A peptide derived from hepatitis C virus comprising an HLA-binding motif in its sequence and being recognized by an antibody detected in a patient with hepatitis C virus infection having the amino acid sequence represented by SEQ ID NO: 2.

2. The peptide derived from hepatitis C virus according to claim 1, wherein the peptide has an amino acid sequence having a homology of at least 70% with the amino acid sequence represented by SEQ ID NO: 2.

3. The peptide derived from hepatitis C virus according to claim 1 or 2, wherein the peptide further has a property of being recognized by an HLA-A24-restricted cytotoxic T cell.

4. A polypeptide comprising a peptide derived from hepatitis C virus according to any one of claims 1 to 3.

5. A polypeptide having an amino acid sequence having a homology of at least 70% with the amino acid sequence of the polypeptide according to claim 4.

6. The polypeptide according to claim 4 or 5, further having a property of being recognized by an HLA-A24-restricted cytotoxic T cell.

7. A nucleotide encoding a peptide derived from hepatitis C virus according to any one of claims 1 to 4 or a polypeptide according to any one of claims 4 to 6, or a nucleotide having a sequence complementary thereto.

8. An antibody or a substance with an antibody-like activity which recognizes a peptide derived from hepatitis C virus according to any one of claims 1 to 3 or a polypeptide according to any one of claims 4 to 6.

9. A vector comprising the nucleotide according to claim 7.

10. An in vitro method of inducing a cytotoxic T cell by using a peptide derived from hepatitis C virus according to any one of claims 1 to 3 or a polypeptide according to any one of claims 4 to 6.

11. An in vitro of detecting a hepatitis virus by using a peptide derived from hepatitis C virus according to any one of claims 1 to 3, a polypeptide according to any one of claims 4 to 6, a nucleotide according to claim 7 or an antibody or a substance with an antibody-like activity according to claim 8.

12. A method of diagnosing hepatitis C virus infection by using a peptide derived from hepatitis C virus according to any one of claims 1 to 3, a polypeptide according to any one of claims 4 to 6, a nucleotide according to claim 7 or an antibody or a substance with an antibody-like activity according to claim 8.

13. The use of a peptide derived from hepatitis C virus according to any one of claims 1 to 3, a polypeptide according to any one of claims 4 to 6, a nucleotide according to claim 7 or an antibody or a substance with an antibody-like activity according to claim 8 for the preparation of a pharmaceutical composition for preventing or treating hepatitis C virus infection.

14. A pharmaceutical composition comprising as an active ingredient a peptide derived from hepatitis C virus according to any one of claims 1 to 3, a polypeptide according to any one of claims 4 to 6, a nucleotide according to claim 7 or an antibody or a substance with an antibody-like activity according to claim 8.

15. The pharmaceutical composition according to claim 14, which is a hepatitis C virus vaccine.

16. A method of predicting the prognosis of hepatitis C virus infection by using a peptide derived from hepatitis C virus according to any one of Claims 1 to 3, a polypeptide according to any one of Claims 4 to 6, a nucleotide according to claim 7 or an antibody or a substance with an antibody-like activity according to claim 8.

17. A kit for diagnosing hepatitis C virus infection or predicting the prognosis of hepatitis C virus infection comprising a peptide derived from hepatitis C virus according to any one of claims 1 to 3, a polypeptide according to any one of claims 4 to 6, a nucleotide according to Claim 7 or an antibody or a substance with an antibody-like activity according to claim 8.


SEQUENCE LISTING
<110>  GREEN PEPTIDE CO., LTD.

<120>  PEPTIDE DERIVED FROM HEPATITIS C VIRUS

<130>  M1562 EP/1

<150>  JP 2003-330258
<151>  2003-09-22

<150>  EP 04 77 3486.8
<151>  2004-09-22

<160>  64

<170>  PatentIn version 3.1

<210>  1
<211>  10
<212>  PRT
<213>  Hepatitis C Virus

<400>  1
Tyr Leu Leu Pro Arg Arg Gly Pro Arg Leu
1               5                   10

<210>  2
<211>  9
<212>  PRT
<213>  Hepatitis C Virus

<400>  2
Arg Tyr Ala Pro Ala Cys Lys Pro Leu
1               5

<210>  3
<211>  9
<212>  PRT
<213>  Hepatitis C Virus

<400>  3
His Tyr Ala Pro Arg Pro Cys Gly Ile
1               5

<210>  4
<211>  9
<212>  PRT
<213>  Hepatitis C Virus

<400>  4
Val Tyr Glu Ala Ala Asp Met Ile Met
1               5

<210>  5
<211>  10
<212>  PRT
<213>  Hepatitis C Virus

<400>  5
Val Tyr His Gly Ala Gly Ser Lys Thr Leu
1               5                   10

<210>  6
<211>  10
<212>  PRT
<213>  Hepatitis C Virus

<400>  6

Ile Phe Leu Leu Ala Leu Leu Ser Cys Leu
1               5                   10

<210>  7
<211>  10
<212>  PRT
<213>  Hepatitis C Virus

<400>  7
Ser Phe Ser Ile Phe Leu Leu Ala Leu Leu
1               5                   10

<210>  8
<211>  9
<212>  PRT
<213>  Hepatitis C Virus

<400>  8
Glu Tyr Val Leu Leu Leu Phe Leu Leu
1               5

<210>  9
<211>  9
<212>  PRT
<213>  Hepatitis C Virus

<400>  9
Met Tyr Thr Asn Val Asp Gln Asp Leu
1               5

<210>  10
<211>  9
<212>  PRT
<213>  Hepatitis C Virus

<400>  10
Gln Tyr Leu Ala Gly Leu Ser Thr Leu
1               5

<210>  11
<211>  9
<212>  PRT
<213>  Hepatitis C Virus

<400>  11
Leu Tyr Gly Val Trp Pro Leu Leu Leu
1               5

<210>  12
<211>  9
<212>  PRT
<213>  Hepatitis C Virus

<400>  12
Ala Tyr Ser Gln Gln Thr Arg Gly Leu
1               5

<210>  13
<211>  9
<212>  PRT
<213>  Hepatitis C Virus

<400>  13
His Tyr Lys Leu Phe Leu Ala Arg Leu
1               5

<210>  14
<211>  9

<212>  PRT
<213>  Hepatitis C Virus

<400>  14
Thr Tyr Ala Thr Tyr Gly Lys Phe Leu
1               5

<210>  15
<211>  9
<212>  PRT
<213>  Hepatitis C Virus

<400>  15
Ala Tyr Met Ser Lys Ala His Gly Ile
1               5

<210>  16
<211>  9
<212>  PRT
<213>  Hepatitis C Virus

<400>  16
Pro Tyr Ile Glu Gln Gly Met Gln Leu
1               5

<210>  17
<211>  9
<212>  PRT
<213>  Hepatitis C Virus

<400>  17
Asn Phe Ile Ser Gly Ile Gln Tyr Leu
1               5

<210>  18
<211>  9
<212>  PRT
<213>  Hepatitis C Virus

<400>  18
Pro Tyr Phe Val Arg Ala His Gly Leu
1               5

<210>  19
<211>  9
<212>  PRT
<213>  Hepatitis C Virus

<400>  19
Gln Phe Lys Gln Lys Ala Ile Gly Leu
1               5

<210>  20
<211>  9
<212>  PRT
<213>  Hepatitis C Virus

<400>  20
Ile Phe Thr Ile Thr Lys Ile Leu Leu
1               5

<210>  21
<211>  10
<212>  PRT
<213>  Hepatitis C Virus

<400>  21
Gly Tyr Ile Pro Ile Val Gly Ala Pro Leu

1                   5                       10

<210>  22
<211>  10
<212>  PRT
<213>  Hepatitis C Virus

<400>  22
Met Tyr Val Gly Gly Val Glu His Arg Leu
1                   5                       10

<210>  23
<211>  10
<212>  PRT
<213>  Hepatitis C Virus

<400>  23
His Tyr Val Gln Met Ala Leu Met Lys Leu
1                   5                       10

<210>  24
<211>  10
<212>  PRT
<213>  Hepatitis C Virus

<400>  24
Thr Tyr Val Tyr Asp His Leu Thr Pro Leu
1                   5                       10

<210>  25
<211>  10
<212>  PRT
<213>  Hepatitis C Virus

<400>  25
Gly Tyr Gly Ala Gly Val Ala Gly Ala Leu
1                   5                       10

<210>  26
<211>  10
<212>  PRT
<213>  Hepatitis C Virus

<400>  26
Ala Tyr Ala Ala Gln Gly Tyr Lys Val Leu
1                   5                       10

<210>  27
<211>  10
<212>  PRT
<213>  Hepatitis C Virus

<400>  27
Ala Tyr Ala Leu Tyr Gly Val Trp Pro Leu
1                   5                       10

<210>  28
<211>  10
<212>  PRT
<213>  Hepatitis C Virus

<400>  28
His Tyr Pro Cys Thr Val Asn Phe Thr Ile
1                   5                       10

<210>  29
<211>  10
<212>  PRT

<213>  Hepatitis C Virus

<400>  29
Tyr Tyr Arg Gly Leu Asp Val Ser Val Ile
1               5                   10

<210>  30
<211>  10
<212>  PRT
<213>  Hepatitis C Virus

<400>  30
Thr Phe Leu Val Gly Leu Asn Gln Tyr Leu
1               5                   10

<210>  31
<211>  10
<212>  PRT
<213>  Hepatitis C Virus

<400>  31
Val Phe Val Gly Leu Ile Leu Leu Thr Leu
1               5                   10

<210>  32
<211>  10
<212>  PRT
<213>  Hepatitis C Virus

<400>  32
Glu Phe Trp Glu Ser Val Phe Thr Gly Leu
1               5                   10

<210>  33
<211>  10
<212>  PRT
<213>  Hepatitis C Virus

<400>  33
Leu Phe Leu Ala Arg Leu Ile Trp Trp Leu
1               5                   10

<210>  34
<211>  10
<212>  PRT
<213>  Hepatitis C Virus

<400>  34
Tyr Phe Ile Thr Arg Ala Glu Ala His Leu
1               5                   10

<210>  35
<211>  10
<212>  PRT
<213>  Hepatitis C Virus

<400>  35
Ala Phe Thr Ala Ser Ile Thr Ser Pro Leu
1               5                   10

<210>  36
<211>  10
<212>  PRT
<213>  Hepatitis C Virus

<400>  36
Asp Phe Lys Thr Trp Leu Gln Ser Lys Leu
1               5                   10

<210>  37
<211>  10
<212>  PRT
<213>  Hepatitis C Virus

<400>  37
Phe Phe Thr Glu Val Asp Gly Val Arg Leu
1               5                   10

<210>  38
<211>  10
<212>  PRT
<213>  Hepatitis C Virus

<400>  38
Ser Phe Ala Ile Lys Trp Glu Tyr Val Leu
1               5                   10

<210>  39
<211>  9
<212>  PRT
<213>  Hepatitis C Virus

<400>  39
Asp Leu Met Gly Tyr Ile Pro Leu Val
1               5

<210>  40
<211>  10
<212>  PRT
<213>  Hepatitis C Virus

<400>  40
Leu Leu Ala Leu Leu Ser Cys Leu Thr Val
1               5                   10

<210>  41
<211>  8
<212>  PRT
<213>  Hepatitis C Virus

<400>  41
Ile Leu His Thr Pro Gly Cys Val
1               5

<210>  42
<211>  9
<212>  PRT
<213>  Hepatitis C Virus

<400>  42
Ser Met Val Gly Asn Trp Ala Lys Val
1               5

<210>  43
<211>  11
<212>  PRT
<213>  Hepatitis C Virus

<400>  43
Ser Leu Leu Ala Pro Gly Ala Lys Gln Asn Val
1               5                   10

<210>  44
<211>  9
<212>  PRT
<213>  Hepatitis C Virus

<400>  44
Cys Ile Asn Gly Val Cys Trp Thr Val
1               5

<210>  45
<211>  9
<212>  PRT
<213>  Hepatitis C Virus

<400>  45
Leu Leu Cys Pro Ala Gly His Ala Val
1               5

<210>  46
<211>  10
<212>  PRT
<213>  Hepatitis C Virus

<400>  46
Lys Leu Val Ala Leu Gly Ile Asn Ala Val
1               5                   10

<210>  47
<211>  10
<212>  PRT
<213>  Hepatitis C Virus

<400>  47
Thr Gly Ala Pro Val Thr Tyr Ser Thr Tyr
1               5                   10

<210>  48
<211>  9
<212>  PRT
<213>  Hepatitis C Virus

<400>  48
Ser Leu Met Ala Phe Thr Ala Ala Val
1               5

<210>  49
<211>  10
<212>  PRT
<213>  Hepatitis C Virus

<400>  49
Leu Leu Phe Asn Ile Leu Gly Gly Trp Val
1               5                   10

<210>  50
<211>  9
<212>  PRT
<213>  Hepatitis C Virus

<400>  50
Ile Leu Ala Gly Tyr Gly Ala Gly Val
1               5

<210>  51
<211>  9
<212>  PRT
<213>  Hepatitis C Virus

<400>  51
Ile Leu Asp Ser Phe Asp Pro Leu Val
1               5

<210> 52
<211> 10
<212> PRT
<213> Hepatitis C Virus

<400> 52
Arg Leu Ile Val Phe Pro Asp Leu Gly Val
1               5                   10

<210> 53
<211> 9
<212> PRT
<213> Hepatitis C Virus

<400> 53
Gly Leu Gln Asp Cys Thr Met Leu Val
1               5

<210> 54
<211> 9
<212> PRT
<213> Hepatitis C Virus

<400> 54
Ala Leu Ala His Gly Val Arg Ala Leu
1               5

<210> 55
<211> 9
<212> PRT
<213> Hepatitis C Virus

<400> 55
Asn Leu Pro Gly Cys Ser Phe Ser Ile
1               5

<210> 56
<211> 9
<212> PRT
<213> Hepatitis C Virus

<400> 56
Ser Tyr Thr Trp Thr Gly Ala Leu Ile
1               5

<210> 57
<211> 9
<212> PRT
<213> Hepatitis C Virus

<400> 57
His Tyr Arg Asp Val Leu Lys Glu Met
1               5

<210> 58
<211> 9
<212> PRT
<213> Hepatitis C Virus

<400> 58
Trp Phe Met Trp Cys Leu Leu Leu Leu
1               5

<210> 59
<211> 9
<212> PRT
<213> Hepatitis C Virus

<400>  59
Ala Phe Tyr Gly Val Trp Pro Leu Leu
1               5

<210>  60
<211>  9
<212>  PRT
<213>  Hepatitis C Virus

<400>  60
Gln Phe Lys Gln Lys Ala Leu Gly Leu
1               5

<210>  61
<211>  10
<212>  PRT
<213>  Hepatitis C Virus

<400>  61
Gln Tyr Ser Pro Gly Gln Arg Val Glu Phe
1               5                   10

<210>  62
<211>  10
<212>  PRT
<213>  Hepatitis C Virus

<400>  62
Gln Phe Lys Gln Lys Ala Leu Gly Leu Leu
1               5                   10

<210>  63
<211>  11
<212>  PRT
<213>  Human Immunodeficiency Virus

<400>  63
Arg Tyr Leu Arg Asp Gln Gln Leu Leu Gly Ile
1               5                   10

<210>  64
<211>  9
<212>  PRT
<213>  Human Immunodeficiency Virus

<400>  64
Ser Leu Tyr Asn Thr Val Ala Thr Leu
1               5